# EUROPEAN PATENT APPLICATION

(11) **EP 3 932 388 A1**
(43) Date of publication of application: **05.01.2022**
(21) Application number: 21170693.2
(22) Date of filing: 27.04.2021
(51) Int. Cl.: A61K 8/98, A61K 33/28, C12M 1/12, C12N 5/0775, A61K 35/28

(54) **METHOD OF PREPARING MESENCHYMAL-LIKE STEM CELLS AND MESENCHYMAL-LIKE STEM CELLS PREPARED THEREBY**

(30) Priority: 30.06.2020 KR 20200080349; 31.07.2020 KR 20200096120; 17.11.2020 KR 20200153819
(71) Applicant: Miraecellbio Co., Ltd., Seoul 04795 (KR); Byon Co., Ltd., Chungcheongbuk-do 28127 (KR)
(72) Inventor: HONG, Ki Sung, 16819 Gyeonggi-do (KR); CHUNG, Hyung Min, 05254 Seoul (KR); KIM, Eun Young, 05501 Seoul (KR); SHIN, Jeong Min, 13512 Gyeonggi-do (KR); KANG, Ah Reum, 13559 Gyeonggi-do (KR)
(74) Representative: Rimini, Rebecca

(57) **Abstract**

Disclosed is a method for effectively preparing mesenchymal-like stem cells, the method including: preparing human pluripotent stem cells cultured to passage 70 or lower after establishment of cell lines; inducing differentiation of the human pluripotent stem cells to produce embryoid bodies and selecting cystic embryoid bodies therefrom; loading the cystic embryoid bodies on a cell-permeable three-dimensional (3D) culture unit to isolate mesenchymal-like stem cells therefrom; isolating only monolayer-shaped cell clusters from the mesenchymal-like stem cells passing through the cell-permeable 3D culture unit; and uniformizing sizes of the monolayer-shaped cell clusters, in which the mesenchymal-like stem cells have anti-inflammatory efficacy and immunosuppression. Further, disclosed are mesenchymal-like stem cells prepared by the preparation method, a transporter or a therapeutic composition including the mesenchymal-like stem cells, and a composition for prevention or treatment of diseases that includes an active ingredient or exosomes secreted from the mesenchymal-like stem cells.

## Description

### CROSS-REFERENCE TO RELATED APPLICATION(S)

This application claims the benefit of Korean Patent Application No. 10-2020-0080349, filed on June 30, 2020, Korean Patent Application No. 10-2020-0096120, filed on July 31, 2020, and Korean Patent Application No. 10-2020-0153819, filed on November 17, 2020, in the Korean Intellectual Property Office, the disclosures of which are incorporated herein by reference.

### BACKGROUND

### 1. Field of the Invention

Example embodiments relate to a method of preparing mesenchymal-like stem cells from human pluripotent stem cells and mesenchymal-like stem cells prepared thereby. Specifically, differentiation of human pluripotent stem cells cultured to passage 70 or lower after establishment of pluripotent cell lines is induced to produce embryoid bodies of various types from which cystic embryoid bodies are selected. The cystic embryoid bodies are loaded on a cell-permeable three-dimensional (3D) culture unit to isolate the mesenchymal-like stem cells therefrom. Only monolayer-shaped cell clusters are isolated from the isolated mesenchymal-like stem cells, and sizes of the monolayer-shaped cell clusters are uniformized to prepare mesenchymal-like stem cells. The high-purity mesenchymal-like stem cells as prepared in this way have anti-inflammatory efficacy and immunosuppression, and express CD90 and SOX2 at a level of 95% or greater. Further, example embodiments relate to mesenchymal-like stem cells prepared by the method, a therapeutic composition containing the mesenchymal-like stem cells or a transporter, and a composition or cosmetic composition for prevention or treatment of diseases that includes an active ingredient or exosomes secreted from the mesenchymal-like stem cells.

### 2. Description of the Related Art

Mesenchyme refers to a tissue of a mesoderm corresponding to an intermediate layer formed by epithelial-mesenchyme transition (EMT) that occurs during embryonic development. Human embryonic stem cells and induced pluripotent stem cells are only cells that may explain such early occurrence in vitro. Human embryonic stem cells and induced pluripotent stem cells may be used as sources of cells with various functions due to their pluripotency.

Currently, studies on human pluripotent stem cells-derived mesenchymal stem cells or mesenchymal progenitor cells as well as adult-derived mesenchymal stem cells are actively being conducted. It was first known that in adults, progenitor cells capable of forming bones in bone marrow like the mesenchymal stem cells exist, and then it has been reported that mesenchymal stem cells may be separated from bone marrow, compact bone, peripheral blood, adipose tissues, and fetal tissues such as umbilical cord blood and an amniotic membrane.

The above adult-derived mesenchymal stem cells have various characteristics that may be useful for cell therapy. Therefore, in actual clinical treatment, the adult-derived mesenchymal stem cells may be applied to a range from a musculoskeletal system such as bone, cartilage and tendon, a cardiovascular system such as myocardial infarction and vascular damage diseases, a respiratory system such as acute and chronic lung damage, and an autoimmune system such as multiple sclerosis, lupus and rheumatoid arthritis to a nervous system such as Lou Gehrig's disease, Parkinson's disease and spinal injury and so on. That is, attempts have been made on cell therapy in various fields.

However, it is known that, unlike embryonic stem cells, general adult mesenchymal stem cells exhibit limited proliferation ability when sub-cultured in vitro for a long period of time, so that a division ability of one cell does not exceed 40 passages. Adult mesenchymal stem cells which are actually applied to cell therapy use only mesenchymal stem cells cultured to passage 7 or lower. Further, since there is no established cell lineage thereof, repetitive cell preparation from adults is required, thereby imposing limitations to the study thereof.

In order to overcome such limitations, studies to isolate and culture mesenchymal stem cells or mesenchymal progenitor cells from human pluripotent stem cells are being conducted. An existing method of obtaining mesenchymal stem cells or progenitor cells from the human pluripotent stem cells includes a method of separating cells representing a desired marker through a flow cytometer (fluorescent activated cell sorter (FACS)) and a method of applying large amounts and large types of cytokines and chemicals in order to induce differentiation thereof.

However, since the method using the flow cytometer uses a laser, not only viability of cells is lowered, but also a culture period is increased due to a small number of cells is obtained after the separation. The method of applying the cytokines and chemicals has a high cost problem due to continuous application and a problem about genetic stability after differentiation and proliferation. Further, it is difficult to effectively control pluripotency of embryonic stem cells.

Separation and proliferation methods of mesenchymal stem cells are very diverse, and approaches to characterization of mesenchymal stem cells are different depending on the origin of tissues and cells. Thus, the International Society for Cell Therapy (ISCT) has proposed three criteria for defining human mesenchymal stem cells. First, the human mesenchymal stem cells need to be adherent when maintained under standard culture conditions. Second, in analyzing surface antigens of the human mesenchymal stem cells, CD90, CD44, CD73, and CD105 need to be expressed at 95% or higher levels, whereas CD45, CD34, CD14 and CD19 need to be expressed at 2% or less level, and expression of undifferentiated control markers Oct3/4, Tra-1-60, Tra-1-81 and immune rejection antigen human leukocyte antigen-antigen D related (HLA-DR) needs to be prevented. Third, the human mesenchymal stem cells need to be differentiated into osteogenic cells, adipogenic cells, and chondrogenic cells in vitro.

Cluster of Differentiation 90 (CD90) as a representative marker defining mesenchymal stem cells is a major factor involved in immune regulation of mesenchymal stem cells. CD90-expressing mesenchymal stem cells are known to upregulate sHLA-G and IL-10 to inhibit proliferation of peripheral blood mononuclear cells (PBMC) activated by phytohemagglutinin (PHA). A human leukocyte antigen G (HLA-G) is a non-classical MHC class I gene and exists in the form of cell membrane conjugates (HLA-G1, -G2, -G3, -G4) and water-soluble forms (sHLA-G5, -G6, and G7). However, the HLA-G has been reported to exhibit immune tolerogenic functions for innate and adaptive cellular responses that have an influence on cytotoxicity of NK and CD81+ T cells and activity of CD41+ T cells. Currently, the HLA-G is understood as a molecule that plays an important role in immune tolerance. Furthermore, it is known that the HLA-G has a function of modulating decidual mononuclear cells into T helper type 2 (Th2) cytokine profiles through regulation of cytokine secretion. Modulation into Th2 cytokine profiles is associated with suppression of immune function. IL-10 as one of Th2 cytokines has a wide range of biological functions such as anti-inflammatory efficacy and immunosuppression. According to a number of reports, in terms of an association between the HLA-G and IL-10, the IL-10 induces expression of the HLA-G, and the HLA-G stimulates expression of the IL-10.

Sex-determining region Y-box 2 (SOX2) is a well-known key transcription factor in human embryonic stem cells and induced pluripotent stem cells, and plays an important role in maintaining cellular pluripotency. SOX2 is expressed in some adult stem cells. It is known that SOX2 has an important influence on differentiation ability and proliferation of cells through regulation of DKK1 and cMyc that are Wnt signaling soluble inhibitors. Thus, a function of SOX2 expressed in human pluripotent stem cells and a function of SOX2 expressed in mesenchymal stem cells are regarded as being different from each other to function in different roles.

Currently, biological or clinical interest in stem cell therapeutics is continuously increasing mainly due to cell characteristics such as immune suppression and tolerance. Human pluripotent stem cells-derived mesenchymal-like stem cells according to the present disclosure are mesenchymal stem cells characterized by expressing CD90 and SOX2 at a level of 95% or higher, and have not been reported to date. This not only satisfies the interest of the research area, but may be given a sufficient meaning from the perspective of the clinical area.

It is required to develop a method for preparation of mesenchymal stem cells that may increase cell viability to produce high-purity mesenchymal-like stem cells, effectively control human pluripotent stem cells, and maintain genetic stability of the prepared mesenchymal-like stem cells.

### SUMMARY

An aspect provides a method of preparing mesenchymal-like stem cells that meet the minimum requirement of human mesenchymal stem cells regulated by the International Society for Cell Therapy.

Another aspect provides a method for preparing mesenchymal-like stem cells, the method including: (a) preparing human pluripotent stem cells cultured to passage 70 or lower after establishment of cell lines; (b) inducing differentiation of the human pluripotent stem cells to produce embryoid bodies and selecting cystic embryoid bodies therefrom; (c) loading the cystic embryoid bodies on a cell-permeable three-dimensional (3D) culture unit to isolate mesenchymal-like stem cells therefrom; (d) isolating only monolayer-shaped cell clusters from the mesenchymal-like stem cells passing through the cell-permeable 3D culture unit; and (e) uniformizing each of longitudinal and transverse sizes of the monolayer-shaped cell clusters to 100 µm to 500 µm, and culturing the mesenchymal-like stem cells, in which the mesenchymal-like stem cells have anti-inflammatory efficacy and immunosuppression, and express CD90 and SOX2 at a level of 95% or greater. Further, the present disclosure relates to human pluripotent stem cells-derived mesenchymal-like stem cells as prepared by the above preparation method.

However, the aspects to be achieved by the present disclosure is not limited to the aspects mentioned above. Other aspects that are not mentioned will be clearly understood by those of ordinary skill in the art from the following description.

According to example embodiments, by a mesenchymal-like stem cells preparation method, mesenchymal-like stem cells may be separated, cultured and proliferated at high efficiency and high purity. Further, according to the mesenchymal-like stem cells preparation method , novel human pluripotent stem cells-derived mesenchymal-like stem cells which have anti-inflammatory efficacy and immunosuppression, and highly express CD90 and SOX2 at a level of 95% or greater may be prepared from the human pluripotent stem cells.

Mesenchymal-like stem cells prepared by a method of preparing mesenchymal-like stem cells according to an example embodiment may exhibit anti-inflammatory efficacy and immunosuppression.

Mesenchymal-like stem cells prepared by a method of preparing mesenchymal-like stem cells according to an example embodiment may exhibit anti-inflammatory efficacy and immunosuppression according to specific marker expression patterns.

Mesenchymal-like stem cells prepared by a method of preparing mesenchymal-like stem cells according to an example embodiment may express CD90 and SOX2 at a level of 95% or greater, and may simultaneously meet minimal requirements and additional features as human mesenchymal stem cells.

Mesenchymal-like stem cells prepared by a method of preparing mesenchymal-like stem cells according to an example embodiment may be applied to a therapeutic composition or a transporter containing the same.

Active ingredients or exosomes secreted from mesenchymal-like stem cells prepared by a method of preparing mesenchymal-like stem cells according to an example embodiment may be applied to a composition or a cosmetic composition for preventing or treating diseases that contains the same.

However, the effect of the present disclosure is not limited to the above effect. It is to be understood that the present disclosure includes all possible effects deduced from the configurations of the disclosure described in the detailed description or claims.

### BRIEF DESCRIPTION OF THE DRAWINGS

These and/or other aspects, features, and advantages of the disclosure will become apparent and more readily appreciated from the following description of example embodiments, taken in conjunction with the accompanying drawings of which:
FIG. 1 illustrates a method of preparing human pluripotent stem cells-derived mesenchymal-like stem cells according to an example embodiment;
FIG. 2 shows forms of cystic embryoid bodies selected from various forms of embryoid bodies obtained by inducing differentiation of human pluripotent stem cells, during the preparation of mesenchymal-like stem cells derived from the human pluripotent stem cells. The embryoid bodies indicated by the white arrows are cystic embryoid bodies. In the cystic embryoid bodies shown in FIG. 2, the cystic embryoid bodies mean embryoid bodies in which a transparent and bright portion is contained in the embryoid bodies, among various types of embryoid bodies;
FIG. 3 shows a cell-permeable three-dimensional (3D) culture unit used for isolation of human pluripotent stem cells-derived mesenchymal-like stem cells according to an example embodiment. Mesenchymal-like stem cells may be isolated by loading the cystic embryoid bodies on the cell-permeable 3D culture unit. Using the cell-permeable 3D culture unit, the mesenchymal-like stem cells may be separated from the selected cystic embryoid bodies at high purity;

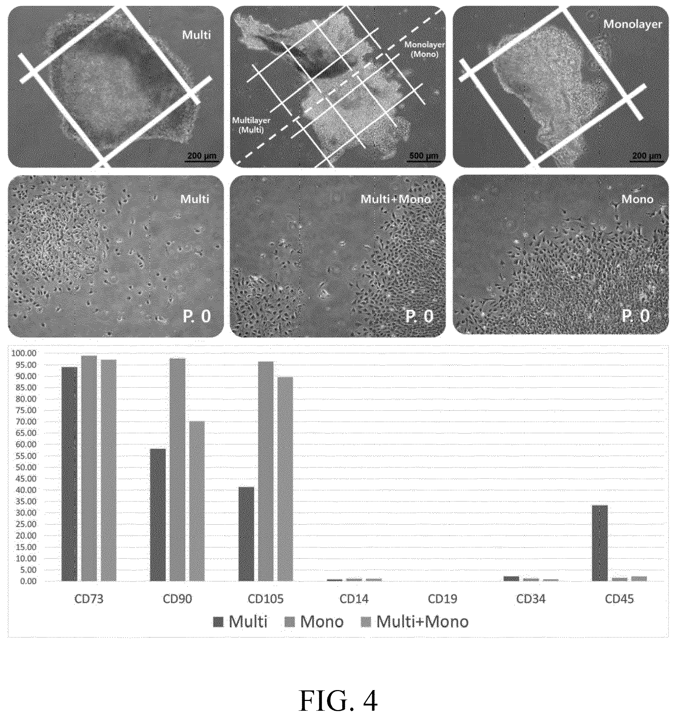

FIG. 4 shows an image of a cluster of mesenchymal-like stem cells initially isolated while the mesenchymal-like stem cells pass through the cell-permeable 3D culture unit according to an example embodiment. In addition, the graph is a comparative measurement of cell surface antigen expressions of initially isolated clusters while attaching each of the initially isolated clusters to a dish (P0). The cluster of mesenchymal-like stem cells initially isolated while the mesenchymal-like stem cells pass through the cell-permeable 3D culture unit may include any one or more of a multilayer shape or a monolayer shape. However, in an example embodiment, only monolayer-shaped cell clusters are separated and cultured;
FIG. 5 is an image obtained by photographing a shape of mesenchymal-like stem cells according to an example embodiment;

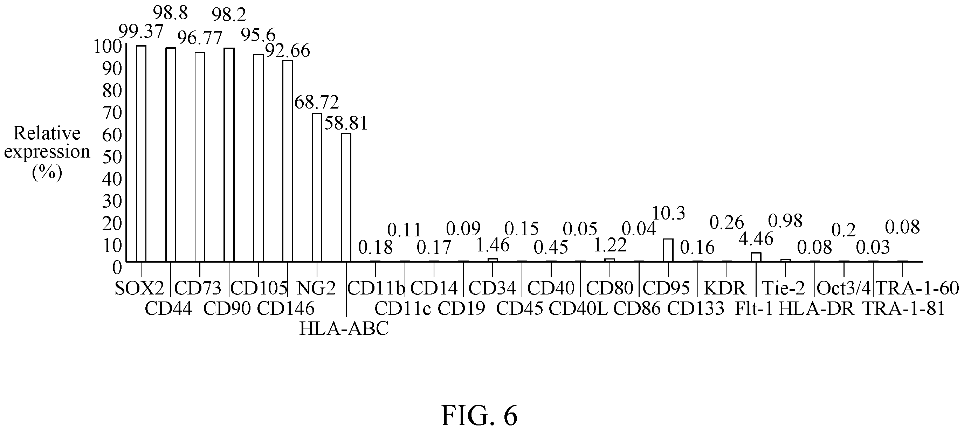

FIG. 6 is a graph measuring expressions of various surface antigens of mesenchymal-like stem cells according to an example embodiment. The mesenchymal-like stem cells according to the present disclosure highly express CD90 at 98.2%, CD44 at 98.8%, CD73 at 96.77% and CD105 at 95.6%. The mesenchymal-like stem cells according to the present disclosure have low expressions of 0.15% of CD45, 1.46% of CD34, 0.17% of CD14 and 0.09% of CD19, and 0.2% of Oct3/4, 0.08% of Tra-1-60, and 0.03% of Tra-1-81. The mesenchymal-like stem cells according to the present disclosure hardly express HLA-DR at 0.08%. Therefore, mesenchymal-like stem cells according to the present disclosure are novel mesenchymal stem cells that meet the minimum standards for human mesenchymal stem cells as determined by the World Stem Cell Therapy Association. The mesenchymal-like stem cells according to the present disclosure express CD90 at a higher level and at the same time, express SOX2 at 99.37%;
FIG. 7 illustrates images of differentiation characteristics of mesenchymal-like stem cells according to an example embodiment into adipogenic cells, osteogenic cell, chondrogenic cells and myogenic cells, as stained with Oil Red O staining, Alizarin red S staining, Alcian blue staining, and immunocytochemistry staining;
FIG. 8 is a graph of comparison results between proliferative capacity (cumulative PDL (cPDL)) and cell sizes of mesenchymal-like stem cells according to an example embodiment, and the proliferative capacity and cell sizes of bone marrow-derived mesenchymal stem cells (BM-MSC);
FIG. 9 is a graph showing that mesenchymal-like stem cells according to an example embodiment are separated at high purity. It may be identified that 99% or more of Oct4 as an undifferentiated regulatory marker has been removed from mesenchymal-like stem cells (MMSC);
FIGS. 10A and 10B show that genetic stability of mesenchymal-like stem cells according to an example embodiment was identified through GTG-banding and single nucleotide polymorphisms (SNP) analysis;
FIG. 11 is a graph and an image showing a tissue regeneration ability of mesenchymal-like stem cells according to an example embodiment based on a migration rate of a cell. In the graph and image, the tissue regeneration ability of mesenchymal-like stem cells according to an example embodiment is compared to bone marrow-derived mesenchymal stem cells (BM-MSCs);
FIG. 12 shows an exchange-of-materials ability of mesenchymal-like stem cells according to an example embodiment with human umbilical vein endothelial cells (HUVEC) when co-culturing the former and latter;
FIG. 13 shows a direct angiogenesis ability of mesenchymal-like stem cells according to an example embodiment;
FIG. 14 is a graph showing a comparison of immunosuppression and anti-inflammatory efficacy between mesenchymal-like stem cells according to an example embodiment and BM-MSCs;
FIG. 15 is a graph showing a comparison between the mesenchymal-like stem cells according to an example embodiment and BM-MSCs in terms of a cell survival ability, a tissue regeneration ability, and a release ability of apoptosis-inhibiting functional substance;
FIG. 16 shows genes related to inflammation regulation which are expressed from mesenchymal-like stem cells according to an example embodiment at a higher level than from BM-MSCs;
FIG. 17 shows genes related to immunosuppression which are expressed from mesenchymal-like stem cells according to an example embodiment at a higher level than from BM-MSCs;
FIG. 18 shows analysis results of cell lysate proteomes of mesenchymal-like stem cells according to an example embodiment which are expressed from mesenchymal-like stem cells according to an example embodiment at a higher level than from BM-MSCs;
FIG. 19 shows analysis results of cell lysate proteomes of mesenchymal-like stem cells according to an example embodiment which are expressed from mesenchymal-like stem cells according to an example embodiment at a lower level than from BM-MSCs;
FIG. 20 shows analysis results of culture supernatant proteomes of mesenchymal-like stem cells according to an example embodiment which are expressed from mesenchymal-like stem cells according to an example embodiment at a higher level than from BM-MSCs;
FIG. 21 shows analysis results of culture supernatant proteomes of mesenchymal-like stem cells according to an example embodiment which are expressed from mesenchymal-like stem cells according to an example embodiment at a lower level than from BM-MSCs;
FIG. 22 shows decrease in the expression of surface antigens of mesenchymal-like stem cells when the number of passages of embryonic stem cells (hESC) exceeds 70 (P68+7 or P68+15). In particular, as the number of passages of embryonic stem cells exceeds 70, the expression of CD90 as a surface antigen of mesenchymal-like stem cells is significantly reduced; and
FIG. 23 shows results of PCR electrophoresis of gene expression of adipogenic cells and osteoblastic cells obtained by inducing of differentiation of mesenchymal-like stem cells isolated according to the number of passages (a(P68), b(P68+7), c(P68+15)) of embryonic stem cells (hESC).

### DETAILED DESCRIPTION

Hereinafter, example embodiments will be described in detail with reference to the accompanying drawings. However, since various changes may be made to the example embodiments, the scope of the rights of the patent application is not limited or limited by these example embodiments. It should be understood that all modifications, equivalents or substitutes to the example embodiments are included within the scope of the rights of the patent application.

The terminology used herein is for the purpose of describing particular embodiments only and is not intended to be limiting of example embodiments. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms including technical and scientific terms used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

When describing the example embodiments with reference to the accompanying drawings, like reference numerals refer to like constituent elements and a repeated description related thereto will be omitted. In the description of example embodiments, detailed description of well-known related structures or functions will be omitted when it is deemed that such description will cause ambiguous interpretation of the present disclosure.

Also, the terms "first," "second," "A," "B," "(a)," "(b)," and the like may be used herein to describe components according to example embodiments. Each of these terminologies is not used to define an essence, order or sequence of a corresponding component but used merely to distinguish the corresponding component from other component(s).

A component having a common function with a component included in an example embodiment is described using a like name in another example embodiment. Unless otherwise described, description made in an example embodiment may be applicable to another example embodiment and detailed description within a duplicate range is omitted.

According to one aspect, there is provided a method of preparing mesenchymal-like stem cells, the method including: (a) preparing human pluripotent stem cells cultured to passage 70 or lower after establishment of cell lines; (b) inducing differentiation of the human pluripotent stem cells to produce embryoid bodies and selecting cystic embryoid bodies therefrom; (c) loading the cystic embryoid bodies on a cell-permeable three-dimensional (3D) culture unit to isolate mesenchymal-like stem cells therefrom; (d) isolating only monolayer-shaped cell clusters from the mesenchymal-like stem cells passing through the cell-permeable 3D culture unit; and (e) uniformizing each of longitudinal and transverse sizes of the monolayer-shaped cell clusters to 100 µm to 500 µm, and culturing the mesenchymal-like stem cells, in which the mesenchymal-like stem cells have anti-inflammatory efficacy and immunosuppression, and express CD90 and SOX2 at a level of 95% or greater.

According to an example embodiment, the cell-permeable 3D culture unit may be made of any one or more selected from a group consisting of nylon, fiber, polyethylene, polypropylene, graphene, titanium, copper, nickel, silver, gold, and platinum.

According to another aspect, there are provided human pluripotent stem cells-derived mesenchymal-like stem cells prepared by the method for preparing mesenchymal-like stem cells, in which the mesenchymal-like stem cells express matrix metalloproteinase-1 (MMP-1) protein and HGF protein at a higher level than bone marrow-derived mesenchymal stem cells express MMP-1 protein and HGF protein, and express CD95 at a lower level than the bone marrow-derived mesenchymal stem cells express CD95, and express CD90 and SOX2 at a level of 95% or greater.

According to an example embodiment, the mesenchymal-like stem cells may express the MMP-1 protein at a level higher by 15 times or greater than bone marrow-derived mesenchymal stem cells express the MMP-1 protein, and express HGF protein at a level higher by 2 times or greater than bone marrow-derived mesenchymal stem cells express HGF protein.

According to an example embodiment, the mesenchymal-like stem cells may express CD95 at a level lower by 15 times or greater than bone marrow-derived mesenchymal stem cells express CD95.

According to an example embodiment, the mesenchymal-like stem cells may express inflammation-regulating genes at a level higher by 2 times to 100 times or greater than bone marrow-derived mesenchymal stem cells express the same.

The inflammation regulating gene may be any one or more selected from a group consisting of Gata3, Adora2a, Gps2, Psmal, Pbk, Lrfn5, Cdh5, Apoe, Foxfl, Tek, Cx3cl1, Ptger4, Acp5, Bcr, Socs5, and Mdk.

According to an example embodiment, the mesenchymal-like stem cells may simultaneously express Gata3, Adora2a and Gps2 genes.

According to an example embodiment, the mesenchymal-like stem cells may express the immunosuppression gene at a level higher by 2 times to 100 times or greater than bone marrow-derived mesenchymal stem cells express the same. The immunosuppression gene may be any one or more selected from a group consisting of Gata3, Gps2, Psmal, Apoe, Foxfl, Tek, Cx3cl1, Ptger4, Bcr, Socs5, and Mdk.

According to an example embodiment, the mesenchymal-like stem cells may simultaneously express Gata3, Gps2 and Psmal genes.

According to another aspect, there is provided a therapeutic composition containing human pluripotent stem cells-derived mesenchymal-like stem cells prepared by the method for preparing mesenchymal-like stem cells, in which the therapeutic composition may be any one of a cellular therapeutic composition, a cellular gene therapeutic composition, a tissue engineering therapeutic composition, an anti-inflammatory therapeutic composition, an immune therapeutic composition, and a composition for preventing or treating cancer.

According to an example embodiment, the therapeutic composition may further include an active ingredient or exosomes secreted from the mesenchymal-like stem cells.

According to an example embodiment, the therapeutic composition may prevent or treat at least one disease selected from a group consisting of multiple sclerosis, systemic sclerosis, acute myocardial infarction, chronic myocardial infarction, chronic lung disease, acute lung disease, Crohn's disease, fecal incontinence, graft versus host disease, lower extremity ischemia disease, Burger's disease, foot ulcer, lupus, rheumatoid arthritis, acute and chronic pyelitis, inflammatory cystitis, interstitial cystitis, underactive bladder, overactive bladder, frozen shoulder, rotator cuff injury and rupture, musculoskeletal injury caused by various movements, knee cartilage injury, tinnitus, atopic dermatitis, psoriasis, skin damage from burns, skin damage from ultraviolet light, and inflammatory and immune system diseases including retinopathy, ischemic dementia, Alzheimer's dementia, spinal cord injury, Parkinson's disease, and central nervous system disease.

According to another aspect, there is provided a transporter containing human pluripotent stem cells-derived mesenchymal-like stem cells prepared by the method for preparing mesenchymal-like stem cells, in which the transporter carries a pharmaceutical composition therein.

According to another aspect, there is provided a composition for preventing or treating a disease, in which the composition may contain an active ingredient or exosomes secreted from human pluripotent stem cells-derived mesenchymal-like stem cells prepared by the method for preparing mesenchymal-like stem cells.

According to an example embodiment, the disease may include at least one selected from a group consisting of multiple sclerosis, systemic sclerosis, acute myocardial infarction, chronic myocardial infarction, chronic lung disease, acute lung disease, Crohn's disease, fecal incontinence, graft versus host disease, lower extremity ischemia disease, Burger's disease, foot ulcer, lupus, rheumatoid arthritis, acute and chronic pyelitis, inflammatory cystitis, interstitial cystitis, underactive bladder, overactive bladder, frozen shoulder, rotator cuff injury and rupture, musculoskeletal injury caused by various movements, knee cartilage injury, tinnitus, atopic dermatitis, psoriasis, skin damage from burns, skin damage from ultraviolet light, and inflammatory and immune system diseases including retinopathy, ischemic dementia, Alzheimer's dementia, spinal cord injury, Parkinson's disease, and central nervous system disease.

According to another aspect, there is provided a cosmetic composition including an active ingredient or exosomes secreted from human pluripotent stem cells-derived mesenchymal-like stem cells prepared by the method for preparing mesenchymal-like stem cells.

### Preparation of Mesenchymal-like Stem Cells with Enhanced Anti-Inflammatory Efficacy and Immunosuppression

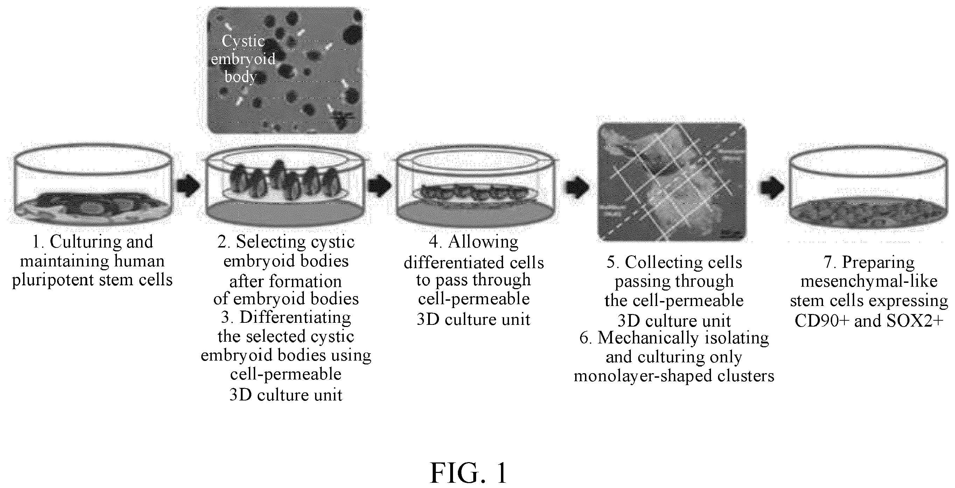

FIG. 1 shows a method of preparing human pluripotent stem cells-derived mesenchymal-like stem cells, the method including: (a) preparing human pluripotent stem cells cultured to passage 70 or lower after establishment of cell lines; (b) inducing differentiation of the human pluripotent stem cells to produce embryoid bodies and selecting cystic embryoid bodies therefrom; (c) loading the cystic embryoid bodies on a cell-permeable 3D culture unit to isolate mesenchymal-like stem cells therefrom; (d) isolating only monolayer-shaped cell clusters from the mesenchymal-like stem cells passing through the cell-permeable 3D culture unit; and (e) uniformizing each of longitudinal and transverse sizes of the monolayer-shaped cell clusters to 100 µm to 500 µm, and culturing the mesenchymal-like stem cells, in which the mesenchymal-like stem cells have anti-inflammatory efficacy and immunosuppression, and express CD90 and SOX2 at a level of 95% or greater.

The first image in FIG. 1 shows the step of culturing and maintaining human pluripotent stem cells. The human pluripotent stem cells are cultured to passage 70 or lower after establishment of the cell line. The second image in FIG. 1 shows the step of formation of embryoid bodies (EB) and then selecting cystic embryoid bodies therefrom, and differentiating the selected cystic embryoid bodies using a cell-permeable 3D culture unit. The selected cystic embryoid bodies are loaded onto the cell-permeable 3D culture unit and pass through the cell-permeable 3D culture unit. The third image in FIG. 1 shows that cystic embryoid bodies pass through the cell-permeable 3D culture unit and move to a bottom of the cell-permeable 3D culture unit. The fourth image in FIG. 1 shows the step of collecting cells that have passed through the cell-permeable 3D culture unit and the step of uniformizing the sizes of the clusters. The cluster in the form of a multilayer is mechanically removed from the collected cell clusters, such that only a monolayer-shaped cluster is cut into a uniform size using a micropipette tip and then selectively cultured. The fifth image in FIG. 1 shows the establishment of mesenchymal-like stem cells expressing CD90+ and SOX2+.

As used herein, "human pluripotent stem cells" are cells in an undifferentiated state and refer to stem cells capable of differentiating into all cells constituting the human body. The human pluripotent stem cells may be any one or more of human embryonic stem cells (hESC), human pluripotent stem cells via somatic cell nuclear transfer (SCNT-hPSC) and induced pluripotent stem cells (iPSC).

The term "mesenchymal-like stem cells" as used herein refers to multipotent mesenchymal stem cells (MMSC) that may differentiate into various cells including bone, cartilage, fat, and muscle cells. In other words, the "mesenchymal-like stem cells" are cells that have functions similar to mesenchymal stem cells (MSCs) present in the matrix, cartilage, bone tissue, and adipose tissue of bone marrow differentiated from the mesoderm via the division of fertilized eggs. That is, mesenchymal-like stem cells according to the present disclosure refer to novel mesenchymal stem cells which express CD90 and SOX2 at higher levels, thereby improving immunosuppression and anti-inflammatory efficacy, or pluripotency retention. Therefore, in the present specification, the expressions "mesenchymal-like stem cells" and "mesenchymal stem cells" are used separately from each other.

In the present specification, n passages or passage n (passage n or P n) means cells obtained by sub-culturing a parental cell line n times. For example, passage 70 or P 70 refers to a cell line obtained by sub-culturing the parent cell line 70 times. n is an integer.

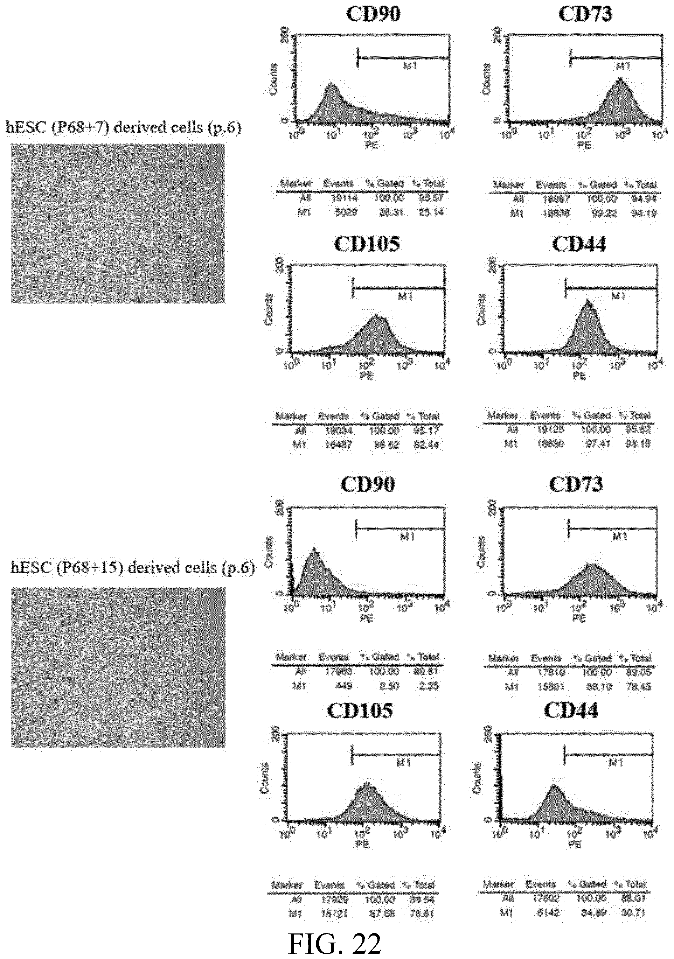

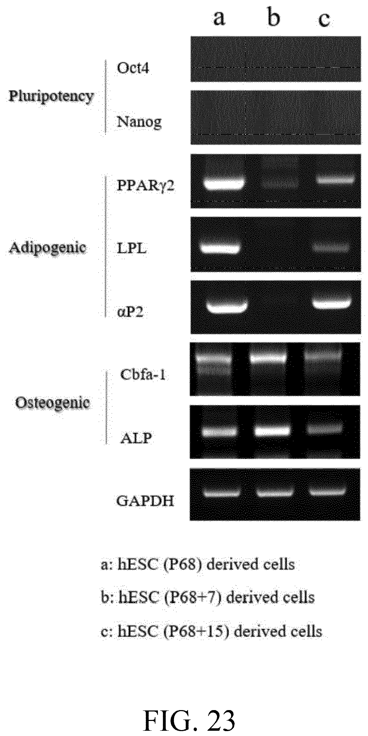

The human pluripotent stem cells used for preparing the mesenchymal-like stem cells according to the present disclosure are characterized by being sub-cultured to passages less than or equal to 70 passages after establishment of the pluripotent cell line. Typically, in the preparation of mesenchymal stem cells using pluripotent stem cells, the pluripotent stem cells are used as they are without passage restrictions. When pluripotent stem cells are used as they are without passage restrictions, most of the surface antigens of prepared mesenchymal stem cells are under-expressed at a non-constant level, and the differentiation efficiency is very low. Furthermore, the prepared mesenchymal stem cells may not express CD90 or SOX2 at 95% level or greater. However, when using human pluripotent stem cells of passage 70 or lower according to the present disclosure, all surface antigens of the prepared mesenchymal-like stem cells were highly expressed at 95% level or greater at a constant level. In particular, mesenchymal-like stem cells expressing each of CD90 and SOX2 at 95% level or greater may be prepared. According to FIG. 22, when the number of passages of pluripotent stem cells (e.g., embryonic stem cells (hESC)) exceeds 70 (P68+7 or P68+15), an amount of expression of CD90 among the surface antigens of mesenchymal-like stem cells is rapidly decreased. That is, as the number of passages increases beyond passage 68, the expression of CD90 decreases to 25% or lower, and CD44, CD73 and CD105 are expressed at 90% level or lower. In addition, when the number of passages of pluripotent stem cells exceeds 70 (P68+7 or P68+15), the expression of CD44, CD73, and CD105 in the mesenchymal-like stem cells is irregular. On the other hand, mesenchymal-like stem cells prepared from pluripotent stem cells sub-cultured no more than 70 times highly expressed CD90 at 98.2% level, 9 CD44 at 98.8% level, CD73 at 96.77% level and CD105 at 95.6% level (see FIG. 6). In FIG. 23, a, b, and c show the results of PCR electrophoresis of gene expression in osteoblastic cells and adipogenic cells obtained by inducing differentiation of mesenchymal stem cells isolated from pluripotent stem cells (e.g., embryonic stem cells (hESC)) at P68, P68+7, and P68+15, respectively. As shown in FIG. 23, in a (P68) where the number of passages is 70 or lower, the mesenchymal stem cells have excellent differentiation ability into adipogenic and osteoblastic cells. On the other hand, in b (P68+7) and c (P68+15) in which the number of passages exceeds 70, the differentiation efficiency of mesenchymal stem cells into adipogenic cells and chondrogenic cells decreases. In other words, mesenchymal-like stem cells prepared using pluripotent stem cells of passage 70 or lower may maintain proliferative ability, differentiation ability, genetic stability, tissue regeneration ability, substance exchange ability, angiogenesis ability, immunosuppressive ability and anti-inflammatory effect which are superior to those of bone marrow-derived mesenchymal stem cells. In addition, mesenchymal-like stem cells prepared using pluripotent stem cells of passage 70 or lower may maintain cell survival, tissue regeneration, and secretion of apoptosis inhibitory functional substances which are superior to those of bone marrow-derived mesenchymal stem cells.

More preferably, when using pluripotent stem cells of passage 70 or lower, and removing the multilayer-shaped cluster from the differentiated cell clusters that have passed through the cell-permeable 3D culture unit, and rather selecting only the monolayer-shaped clusters and mechanically uniformizing the sizes thereof and culturing the same, the surface antigen of the resulting mesenchymal stem cells is expressed at 95% level or greater. In particular, mesenchymal-like stem cells expressing each of CD90 and SOX2 among the surface antigens of mesenchymal stem cells at 95% level or greater may be prepared.

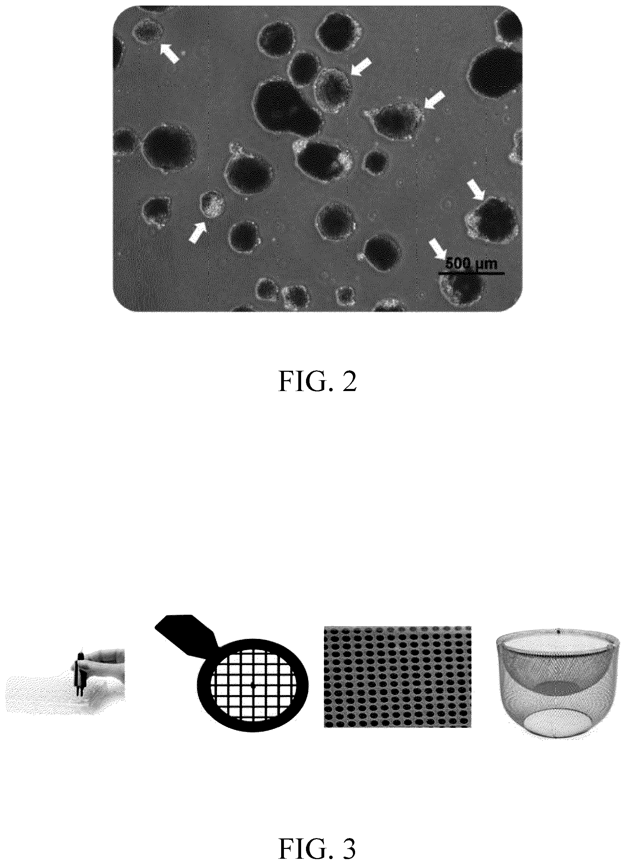

FIG. 2 shows the shape of cystic embryoid bodies used in the preparation of mesenchymal-like stem cells derived from human pluripotent stem cells according to an example embodiment of the present disclosure. Herein, "cystic embryoid bodies" refers to the form of embryoid bodies in which a transparent and bright portion is included in embryoid bodies among various forms of embryoid bodies. Based on this feature, the cystic embryoid bodies may be separated therefrom with the naked eye.

FIG. 3 shows the cell-permeable 3D culture unit used to isolate mesenchymal-like stem cells by loading selected cystic embryoid bodies among embryoid bodies that have been obtained by inducing differentiation of human pluripotent stem cells.

The term "cell-permeable 3D culture unit" as used herein is a cell-permeable instrument. That is, when inducing differentiation culture from embryoid bodies derived from embryonic stem cells, induced pluripotent stem cells, or stem cells via somatic cell nuclear transfer into mesenchymal-like stem cells, the cell-permeable 3D culture unit may allow epithelial-mesenchymal transition (EMT) that occurs during embryonic development to occur naturally. That is, mesenchymal-like stem cells may be separated, cultured and proliferated at high purity and high efficiency using the cell-permeable 3D culture unit.

The cell-permeable 3D culture unit is a cell-permeable artificial insert such as a culture plate for cell culture, a 3D insert for cell culture, or a mesh made of nylon or fibrous material. The cell-permeable 3D culture unit may be prepared using bioprinting technology. The cell-permeable 3D culture unit may be made of one or more of nylon, fiber, polyethylene, polypropylene, graphene, titanium, copper, nickel, silver, gold, and platinum. However, the cell-permeable 3D culture unit is not limited to the material as long as the cell-permeability is guaranteed.

Further, a culture medium for human pluripotent stem cells-derived embryoid body using the cell-permeable 3D culture unit may include EGM2-MV, MCDB, DMEM, MEM-α, STEMPRO-MSC, MesenCult-MSC medium. Desirably, the culture medium may be EGM2-MV, MCDB, DMEM or MEM-α medium. The present disclosure is not limited thereto. The culture medium for human pluripotent stem cells-derived embryoid body using the cell-permeable 3D culture unit may contain one or more additives among fetal bovine serum (FBS), serum replacement (SR), human serum (HS) and human platelet lysate (HPL). Specifically, the additive may be 1 to 20% of FBS, 1 to 20% of SR, 1 to 20% of Human Serum, or 1 to 20% of HPL. Desirably, the additive may be 5% FBS or 2.5 to 5% HPL. The present disclosure is not limited thereto.

FIG. 4 is an image and graph showing the characteristics of each of cell clusters as collected from a bottom of the cell-permeable 3D culture unit after passing through the cell-permeable 3D culture unit in the step of isolating the mesenchymal-like stem cells derived from human pluripotent stem cells. As shown in FIG. 4, the cell cluster collected from the bottom of the cell-permeable 3D culture unit may be a multilayer-shaped cluster or a monolayer-shaped cluster. It is identified based on the results of culturing multilayer-shaped and monolayer-shaped clusters together, or culturing multilayer-shaped and monolayer-shaped clusters, separately that expressions of CD90, CD73, and CD105 as major surface antigens representing mesenchymal stem cells are consistently higher in cells cultured by isolating only the monolayer-shaped clusters.

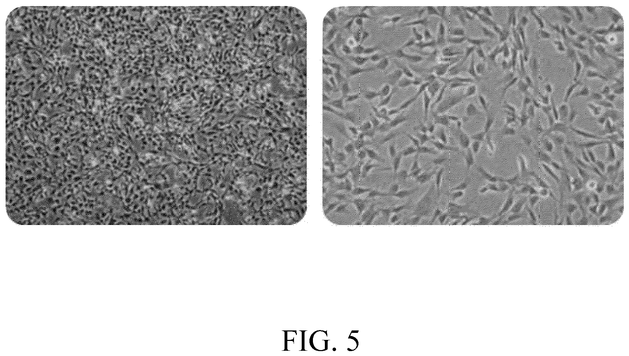

FIG. 5 is an image obtained by photographing a shape of mesenchymal-like stem cells according to an example embodiment.

FIG. 6 is a graph measuring the surface antigen expression of mesenchymal-like stem cells according to an example embodiment. Referring to FIG. 6, the surface antigens CD90, CD44, CD73, CD105, and SOX2 of mesenchymal-like stem cells are expressed at a level of 95% or higher. To the contrary, the surface antigens CD45, CD34, CD14, and CD19 of mesenchymal-like stem cells are expressed at 2% or less level. Further, the undifferentiated regulatory markers Oct3/4, Tra-1-60, Tra-1-81 and the immune rejection antigen HLA-DR are not expressed. Therefore, the mesenchymal-like stem cells according to the present disclosure are mesenchymal stem cells characterized by expressing, at a level higher at a level of 95% or greater, a marker related to immune regulation CD90 and a marker related to maintaining cell pluripotency SOX2. Thus, the mesenchymal-like stem cells according to the present disclosure may be identified as novel mesenchymal stem cells with improved immunosuppression and pluripotency retention.

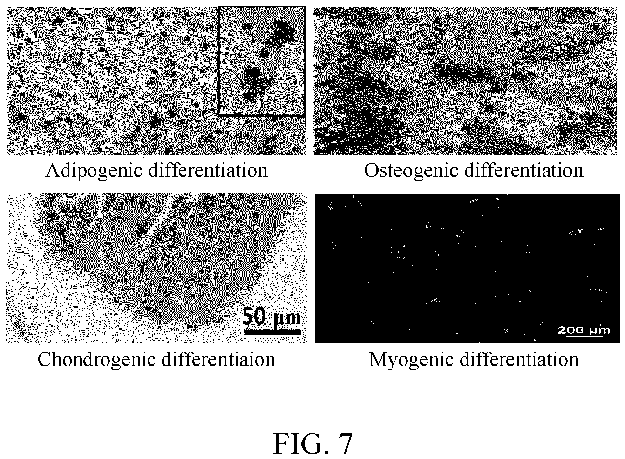

FIG. 7 is a result showing a differentiation ability of mesenchymal-like stem cells according to an example embodiment into various mesodermal cells. Referring to FIG. 7, the mesenchymal-like stem cells may differentiate into adipogenic cells, osteogenic cells, chondrogenic cells, and myogenic cells under an appropriate differentiation environment. This satisfies the requirement for differentiation ability of mesenchymal stem cells defined by the International Society for Cell Therapy.

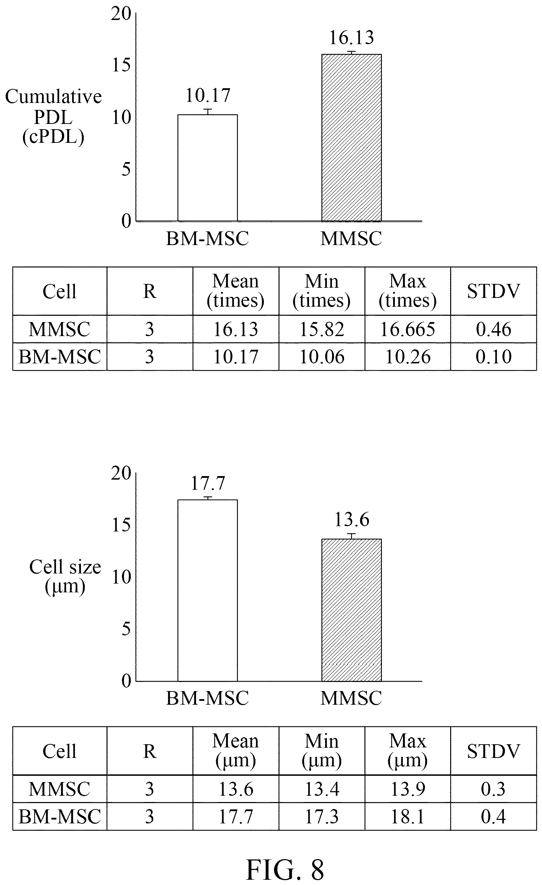

FIG. 8 is a graph comparing between proliferation ability and cell size of mesenchymal-like stem cells according to an example embodiment and those of bone marrow-derived mesenchymal stem cells (bone marrow MSC, BM-MSC). In FIG. 8, cumulative PDL (cPDL) represents cell proliferation ability. Mesenchymal-like stem cells may divide an average of 16.13 times in 4 passages. At this time, the size of mesenchymal-like stem cell is 13.6 µm. The cell proliferation of mesenchymal-like stem cells is about 1.6 times faster, compared to that of bone marrow-derived mesenchymal stem cells. The size of the mesenchymal-like stem cell is identified as being about 0.8 times smaller compared to that of bone marrow-derived mesenchymal stem cell. The mesenchymal-like stem cells have a fast cell proliferation ability and smaller cell size. Thus, when the mesenchymal-like stem cells are used for a stem cell therapeutic composition, the possibility of side effects in the body may be reduced, and therapeutic effects may be improved.

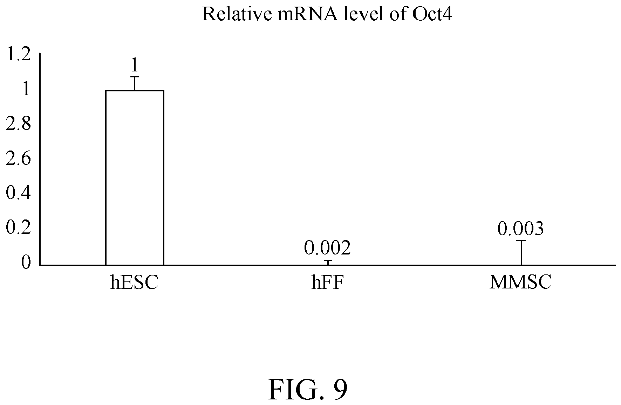

FIG. 9 is a graph showing that mesenchymal-like stem cells according to an example embodiment are separated at high purity. Referring to FIG. 9, mesenchymal-like stem cells in which 99% or higher of Oct4 as an undifferentiated control marker has been removed may be identified. Therefore, when the mesenchymal-like stem cells derived from human pluripotent stem cells sub-cultured to passage 70 or lower after the establishment of the cell line thereof are isolated and cultured according to the preparation method according to the present disclosure, undifferentiated cells other than the mesenchymal-like stem cells are not included. Thus, the mesenchymal-like stem cells may be isolated and cultured at high purity.

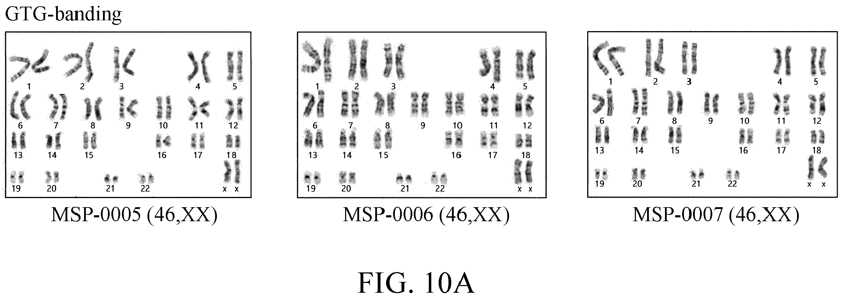

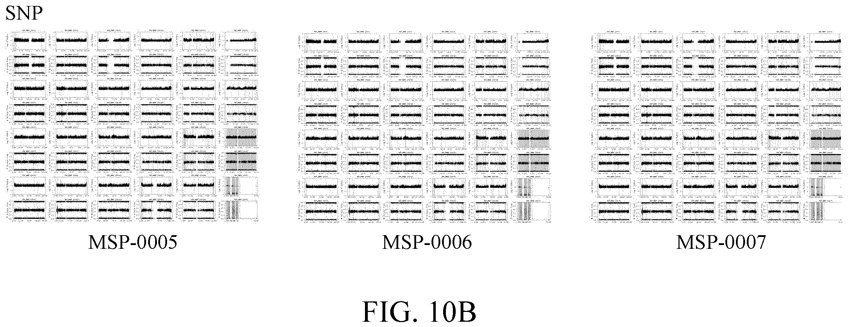

FIGS. 10A and 10B show that genetic stability of mesenchymal-like stem cells according to an example embodiment was identified through GTG-banding and SNP analysis. Referring to FIGS. 10A and 10B, chromosomes are normal in all mesenchymal-like stem cells prepared with three batch numbers, i.e. MSP-0005, MSP-0006, and MSP-0007. Further, it is identified that no SNP abnormality is observed.

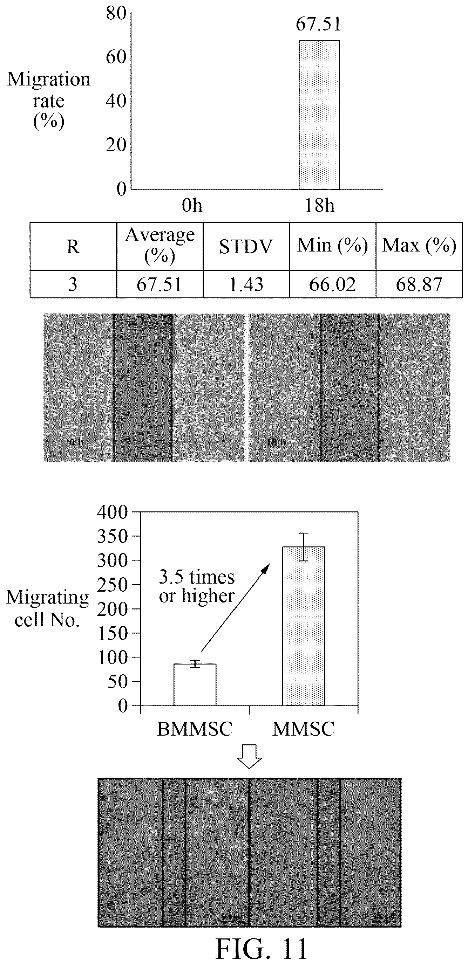

FIG. 11 is a graph identifying a tissue regeneration ability of mesenchymal-like stem cells according to an example embodiment, based on a cell migration rate. Referring to FIG. 11, it was identified based on in vitro wound healing analysis that a tissue regeneration ability of mesenchymal-like stem cells based on the cell migration rate is about 3.5 times or higher than that of bone marrow-derived mesenchymal stem cells, which are adult stem cells.

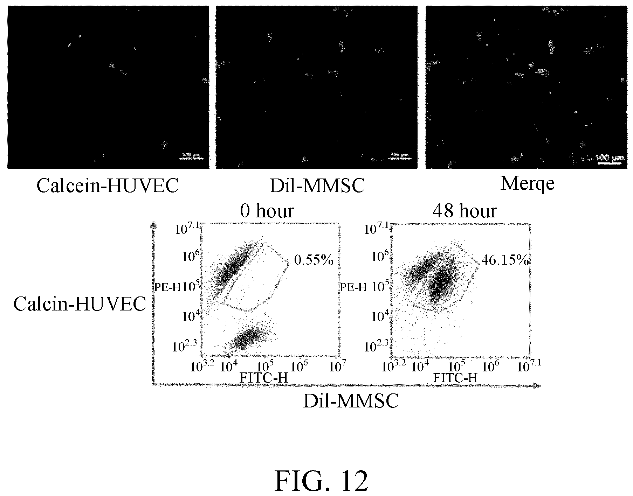

FIG. 12 shows an exchange-of-materials ability of mesenchymal-like stem cells according to an example embodiment. Referring to FIG. 12, an intercellular exchange-of-materials ability of the mesenchymal-like stem cells was identified via co-culture between human umbilical vein endothelial cells (HUVEC) and mesenchymal-like stem cells.

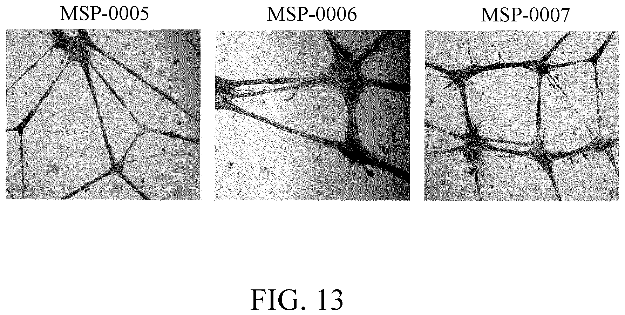

FIG. 13 shows an angiogenesis ability of mesenchymal-like stem cells according to an example embodiment. Referring to FIG. 13, the mesenchymal-like stem cells were cultured on Matrigel to identify spontaneous angiogenesis ability thereof.

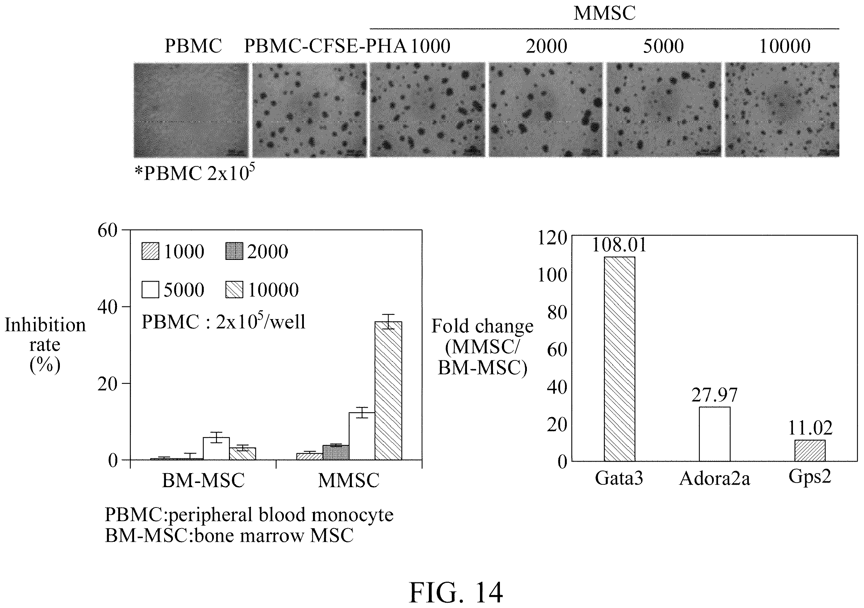

FIG. 14 shows an ability to inhibit immune cell proliferation and anti-inflammatory efficacy of mesenchymal-like stem cells according to an example embodiment. Referring to FIG. 14, a strong anti-proliferative effect of immune cells by mesenchymal-like stem cells was identified via co-culture between monocyte cells as immune cells and the mesenchymal-like stem cells. This indicates that immunosuppression and anti-inflammatory efficacy of mesenchymal-like stem cells are higher than those of bone marrow-derived mesenchymal stem cells.

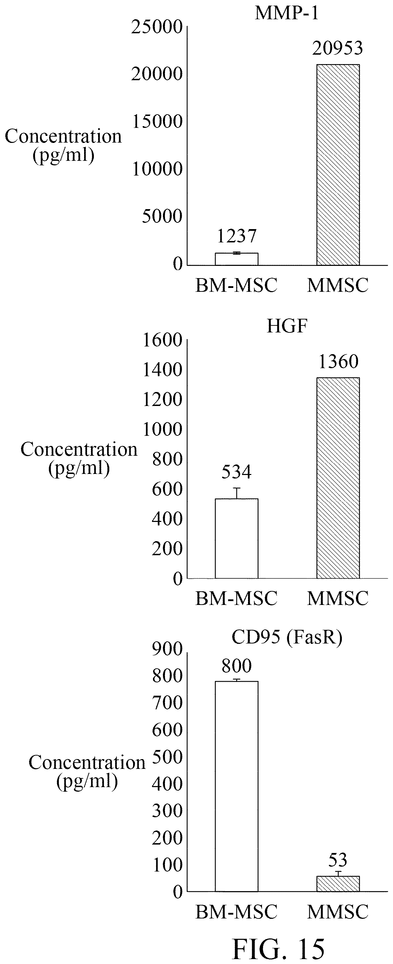

FIG. 15 is a graph showing a cell survival ability, a tissue regeneration ability, and an ability to secrete functional substances that inhibit apoptosis, of the mesenchymal-like stem cells according to an example embodiment.

As shown in FIG. 15, human pluripotent stem cells-derived mesenchymal-like stem cells prepared according to an example embodiment express MMP-1 protein and HGF protein at a higher level than bone marrow-derived mesenchymal stem cells express MMP-1 protein and HGF protein, and which express CD95 at a lower level than the bone marrow-derived mesenchymal stem cells express CD95, and which express CD90 and SOX2 at a level of 95% or greater. Further, according to an example embodiment, the mesenchymal-like stem cells express the MMP-1 protein at a level higher by 15 times or greater than bone marrow-derived mesenchymal stem cells express the MMP-1 protein, and express HGF protein at a level higher by 2 times or greater than bone marrow-derived mesenchymal stem cells express HGF protein. Further, according to an example embodiment, the mesenchymal-like stem cells may express CD95 at a level lower by 15 times or greater than bone marrow-derived mesenchymal stem cells express the same.

As shown in FIG. 15, the MMP-1 protein related to tissue regeneration is secreted from human pluripotent stem cells-derived mesenchymal-like stem cells prepared according to an example embodiment at a level higher by about 16.9 times or greater than from bone marrow-derived mesenchymal stem cells. Further, hepatocyte growth factor (HGF) protein related to cell survival and proliferation is secreted from the mesenchymal-like stem cells at a level higher by about 2.5 times or greater than from bone marrow-derived mesenchymal stem cells. To the contrary, it was identified that Cluster of Differentiation (CD95) related to apoptosis is expressed from the mesenchymal-like stem cells at a level lower by about 15 times or greater than from bone marrow-derived mesenchymal stem cells.

The "Cluster of Differentiation 95 (CD95)" is an apoptosis receptor known as Fas Receptor (FasR), Apoptosis antigen 1 (APO-1), or tumor necrosis factor receptor superfamily member 6 (TNFRSF6). CD95 is located on the cell surface and induces apoptosis by interaction with a ligand known as CD95L (CD95 Ligand). Specifically, when a ligand known as Fas Ligand (FasL) or CD95L (CD95 Ligand) is bound to the CD95, apoptosis is promoted through Death-Inducing Signaling Complex (DISC). That is, when the CD95 is overexpressed in stem cells or progenitor cells, apoptosis signaling via CD95L is activated. To the contrary, stem cells in which CD95 is expressed at a relatively low level may exhibit excellent cell viability due to inhibition of apoptosis signaling.

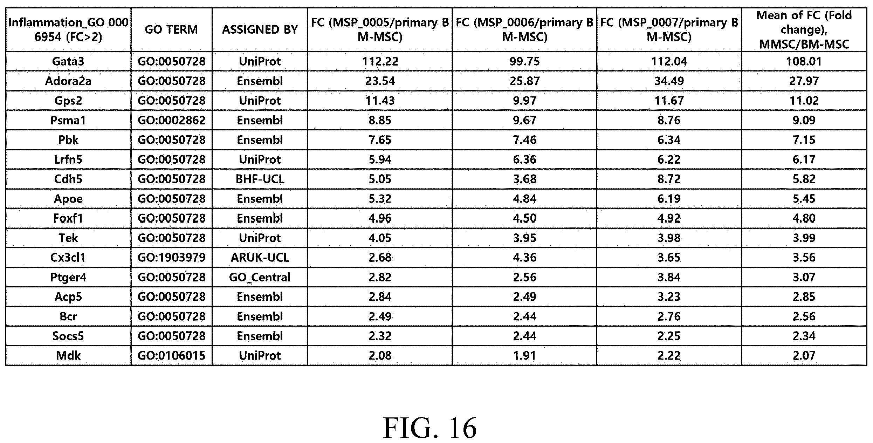

FIG. 16 shows genes related to inflammation regulation that are expressed from human pluripotent stem cells-derived mesenchymal-like stem cells prepared according to an example embodiment at a higher level than from bone marrow-derived mesenchymal stem cells.

As shown in FIG. 16, human pluripotent stem cells-derived mesenchymal-like stem cells prepared according to an example embodiment express inflammation-regulating genes at a level higher by 2 times to 100 times or greater than bone marrow-derived mesenchymal stem cells express the same. The inflammation regulating gene may include at least one selected from a group consisting of Gata3, Adora2a, Gps2, Psmal, Pbk, Lrfn5, Cdh5, Apoe, Foxfl, Tek, Cx3cl1, Ptger4, Acp5, Bcr, Socs5, and Mdk. Further, according to an example embodiment, the mesenchymal-like stem cells that simultaneously express Gata3, Adora2a and Gps2 genes are provided. The inflammation regulating gene is a gene related to inhibition of inflammation. In this connection, the mesenchymal-like stem cells express the inflammation regulating gene at a level higher by 2 times to 100 times or greater than bone marrow-derived mesenchymal stem cells express the same, thereby to enhance the anti-inflammatory efficacy.

As shown in FIG. 16, Gata3 gene is expressed from mesenchymal-like stem cells at a level higher by about 100 times or greater than from bone marrow-derived mesenchymal stem cells. Further, Adora2a and Gps2 genes are expressed from mesenchymal-like stem cells at a level higher by about 10 times or greater than from bone marrow-derived mesenchymal stem cells. Further, Psmal, Pbk, Lrfn5m, Cdh5, and Apoe genes are expressed from mesenchymal-like stem cells at a level higher by about 5 times or greater than from bone marrow-derived mesenchymal stem cells. Further, the mesenchymal-like stem cells may simultaneously express the inflammation regulating genes Gata3, Adora2a and Gps2 gene.

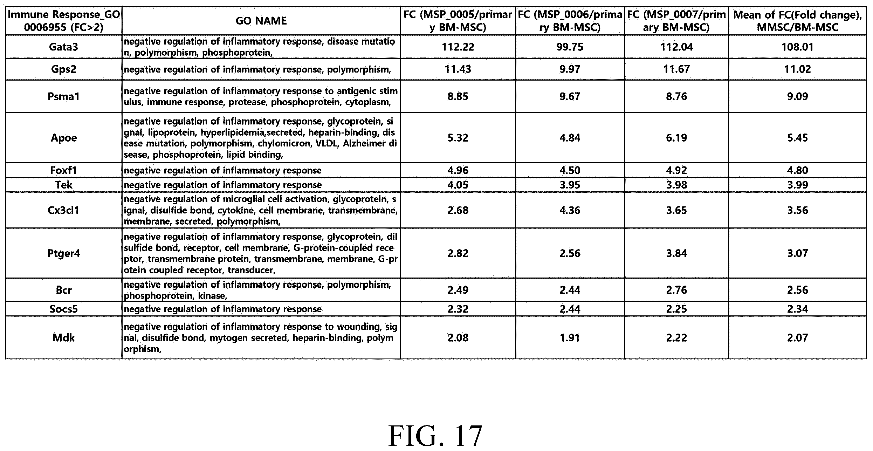

FIG. 17 shows immunosuppression-related genes that are expressed from the human pluripotent stem cells-derived mesenchymal-like stem cells prepared according to an example embodiment at a higher level than from bone marrow-derived mesenchymal stem cells.

As shown in FIG. 17, the mesenchymal-like stem cells express the immunosuppression gene at a level higher by 2 times to 100 times or greater than bone marrow-derived mesenchymal stem cells express the same. The immunosuppression gene may include at least one of Gata3, Gps2, Psmal, Apoe, Foxfl, Tek, Cx3cl1, Ptger4, Bcr, Socs5 and Mdk. Further, the mesenchymal-like stem cells may simultaneously express Gata3, Gps2 and Psmal genes.

As shown in FIG. 17, Gata3 gene is expressed from mesenchymal-like stem cells at a level higher by about 50 times to 100 times or greater than from bone marrow-derived mesenchymal stem cells. Further, the Gps2 gene is expressed from mesenchymal-like stem cells at a level higher by about 5 times to 10 times or greater than from bone marrow-derived mesenchymal stem cells. Further, Psmal and Apoe genes are expressed from mesenchymal-like stem cells at a level higher by about 5 times or greater than from bone marrow-derived mesenchymal stem cells. Further, mesenchymal-like stem cells may simultaneously express the immunosuppression genes Gata3, Gps2 and Psmal.

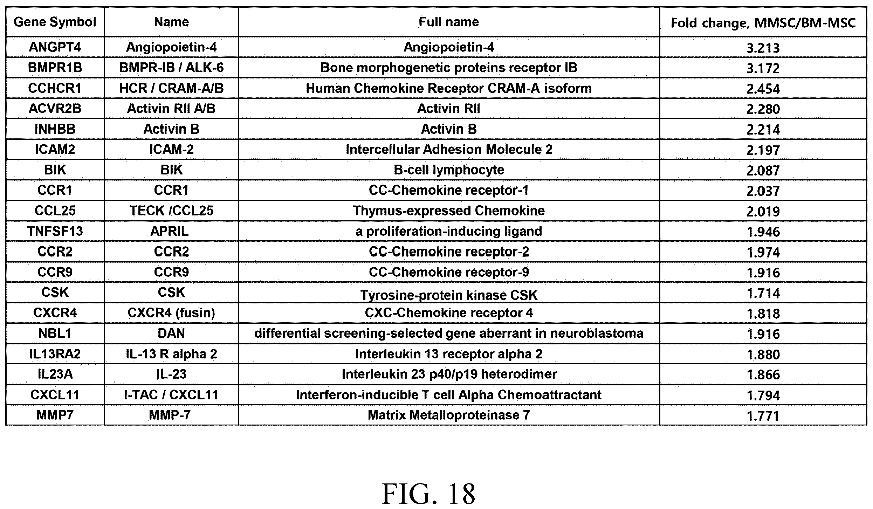

FIG. 18 shows analysis results of cell lysate proteomes of mesenchymal-like stem cells according to an example embodiment which are expressed from mesenchymal-like stem cells according to an example embodiment at a higher level than from bone marrow-derived mesenchymal stem cells. Referring to FIG. 18, mesenchymal-like stem cells express Angiopoietin-4 (ANGPT4), bone morphogenic protein receptor 1B (BMPR-1B), Activin B, Activin RII, CCR1, human chemokine receptor (HCR) CRAM-A isoform, and intracellular adhesion molecule 2 (ICAM- 2) at a level higher by about 2 times or greater than bone marrow-derived mesenchymal stem cells express the same. The mesenchymal-like stem cells may simultaneously express Angiopoietin-4 and BMPR-1B. Desirably, the mesenchymal-like stem cells may express Angiopoietin-4 and BMPR-1B at a level higher by about 3 times or greater than bone marrow-derived mesenchymal stem cells express the same. More desirably, the mesenchymal-like stem cells express any one or more proteomes of ANGPT4, BMPR-1B, Activin B, Activin RII, CCR1, HCR and ICAM-2 at a level higher by about 2 times or greater than the bone marrow-derived mesenchymal stem cells express the same.

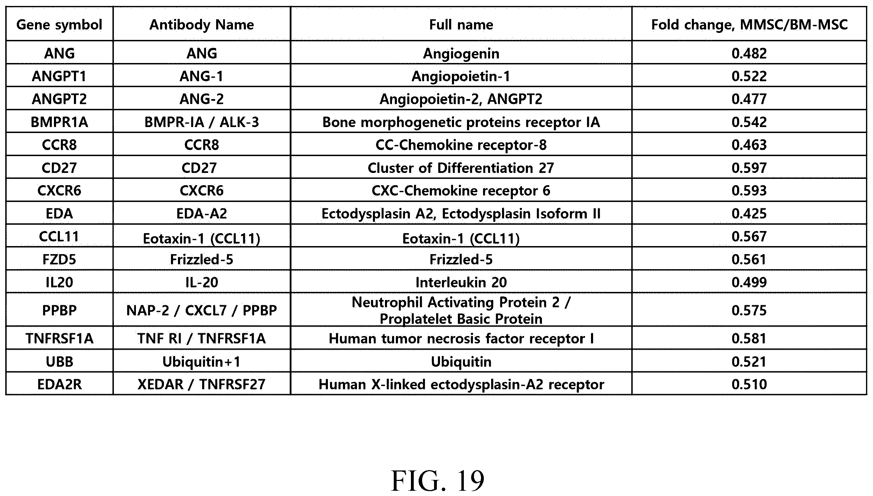

FIG. 19 shows analysis results of the cell lysate proteomes of mesenchymal-like stem cells according to an example embodiment which are expressed from mesenchymal-like stem cells according to an example embodiment at a lower level than from bone marrow-derived mesenchymal stem cells. Referring to FIG. 19, the mesenchymal-like stem cells may secrete Angiogenin, Angiopoietin-2, CCR8, EDA-A2, and IL-20 at a level lower by about two times or greater than bone marrow-derived mesenchymal stem cells express the same. The mesenchymal-like stem cells may simultaneously secrete Angiogenin and Angiopoietin-2. The mesenchymal-like stem cells may secrete Angiogenin and Angiopoietin-2 at a level lower by about two times or greater than bone marrow-derived stem cell express the same.

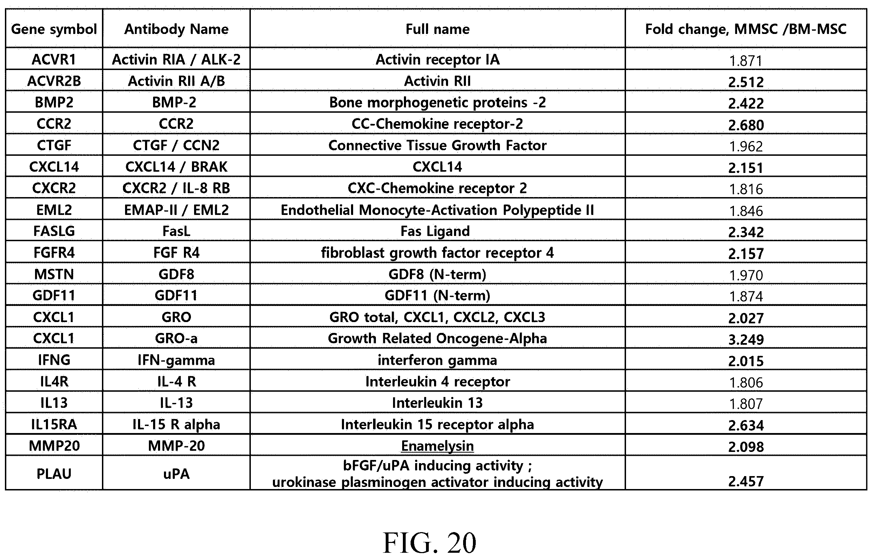

FIG. 20 shows analysis results of the culture supernatant proteomes of mesenchymal-like stem cells according to an example embodiment which are expressed from mesenchymal-like stem cells according to an example embodiment at a higher level than from bone marrow-derived mesenchymal stem cells. Referring to FIG. 20, mesenchymal-like stem cells express GRO-a, IL-15R alpha, FasL, Activin RII, BMP-2, CCR2, CXCL14, FGFR4, uPA, MMP-20 at a level higher by 2 times or greater than bone marrow-derived mesenchymal stem cells express the same. The mesenchymal-like stem cells may simultaneously secrete GRO-a and IL-15R alpha. The mesenchymal-like stem cells express GRO-a and IL-15R alpha at a level higher by 2 times or greater than bone marrow-derived mesenchymal stem cells express the same. The mesenchymal stem cells may secrete any one of GRO-a, IL-15R alpha, FasL, Activin RII, BMP-2, CCR2, CXCL14, and FGFR4 at a level higher by 2 times or greater than bone marrow-derived mesenchymal stem cells express the same.

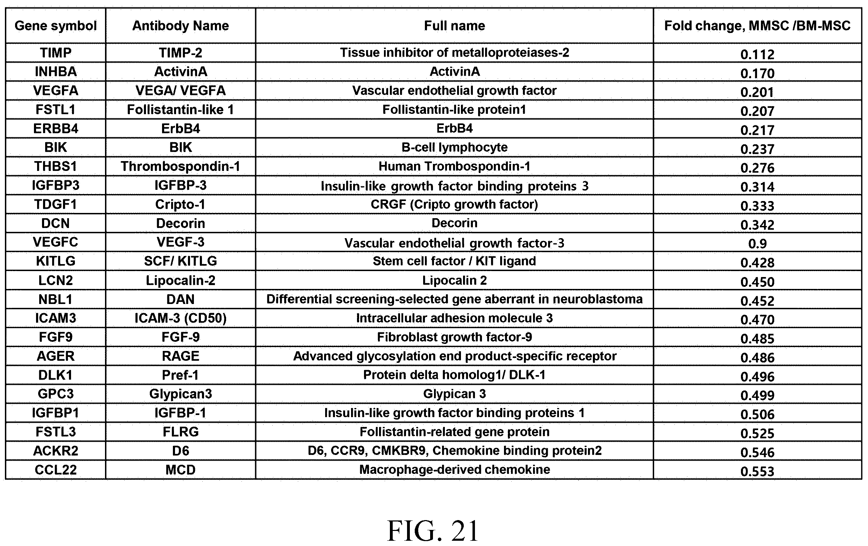

FIG. 21 shows analysis results of the culture supernatant proteomes of mesenchymal-like stem cells according to an example embodiment which are expressed from mesenchymal-like stem cells according to an example embodiment at a lower level than from bone marrow-derived mesenchymal stem cells. Referring to FIG. 21, the mesenchymal-like stem cells secrete TIMP-2, Activin A, VEGF A, Follistantin-like 1, ErbB4m, and Thrombospondin-1 at a level lower by 4 times or greater than bone marrow-derived mesenchymal stem cells express the same. The mesenchymal-like stem cells may secrete TIMP-2 and Activin A at the same time, and may secrete TIMP-2 and Activin A at a level lower by 5 times or greater than bone marrow-derived mesenchymal stem cells express the same.

The mesenchymal-like stem cells according to the present disclosure may inhibit a proliferation ability when co-cultured with human peripheral blood-derived monocyte cells. More specifically, when co-cultured with human peripheral blood-derived monocyte cells, the mesenchymal-like stem cells according to the present disclosure may inhibit proliferation about 5 times to about 8 times or greater than bone marrow-derived mesenchymal stem cells may inhibit.

The mesenchymal-like stem cells according to the present disclosure may exhibit about 3.5 times or higher repair ability than bone marrow-derived mesenchymal stem cells may exhibit, in evaluation of cell migration and repair ability.

The mesenchymal-like stem cells according to the present disclosure may secrete MMP-1 protein as a gene related to cell survival at a level higher by about 15 times to about 16 times or greater than bone marrow-derived mesenchymal stem cells. Further, the mesenchymal-like stem cells may secrete HGF protein as a gene related to cell growth and tissue regeneration at a level higher by about 2 times or greater than bone marrow-derived mesenchymal stem cells.

The mesenchymal-like stem cells according to the present disclosure may express CD95 as an apoptosis receptor at a level lower by about 15 times or greater than the bone marrow-derived mesenchymal stem cells.

A therapeutic composition containing the mesenchymal-like stem cells prepared by the preparation method according to an example embodiment may include any one of a cellular therapeutic composition, a cellular gene therapeutic composition, a tissue engineering therapeutic composition, an anti-inflammatory therapeutic composition, an immune therapeutic composition, and a composition for preventing or treating cancer. Further, the therapeutic composition may further contain an active ingredient or exosomes secreted from the mesenchymal-like stem cells. Further, the therapeutic composition may prevent or treat at least one disease among multiple sclerosis, systemic sclerosis, acute myocardial infarction, chronic myocardial infarction, chronic lung disease, acute lung disease, Crohn's disease, fecal incontinence, graft versus host disease, lower extremity ischemia disease, Burger's disease, foot ulcer, lupus, rheumatoid arthritis, acute and chronic pyelitis, inflammatory cystitis, interstitial cystitis, underactive bladder, overactive bladder, frozen shoulder, rotator cuff injury and rupture, musculoskeletal injury caused by various movements, knee cartilage injury, tinnitus, atopic dermatitis, psoriasis, skin damage from burns, skin damage from ultraviolet light, and inflammatory and immune system diseases including retinopathy, ischemic dementia, Alzheimer's dementia, spinal cord injury, Parkinson's disease, and central nervous system disease.

The active ingredients or exosomes secreted from the mesenchymal-like stem cells may include all functional substances related to immunosuppression, anti-inflammatory efficacy, cell survival, tissue regeneration, or inhibition of apoptosis. However, as long as the therapeutic composition has a material having functionality, the material is not limited thereto.

A transporter containing mesenchymal-like stem cells prepared by the preparation method according to an example embodiment may be provided. The transporter may be characterized by carrying a pharmaceutical composition therein.

A composition for preventing or treating a disease may be provided. The composition may contain active ingredients or exosomes secreted from the mesenchymal-like stem cells prepared by the preparation method according to an example embodiment. In this connection, the disease may include at least one of multiple sclerosis, systemic sclerosis, acute myocardial infarction, chronic myocardial infarction, chronic lung disease, acute lung disease, Crohn's disease, fecal incontinence, graft versus host disease, lower extremity ischemia disease, Burger's disease, foot ulcer, lupus, rheumatoid arthritis, acute and chronic pyelitis, inflammatory cystitis, interstitial cystitis, underactive bladder, overactive bladder, frozen shoulder, rotator cuff injury and rupture, musculoskeletal injury caused by various movements, knee cartilage injury, tinnitus, atopic dermatitis, psoriasis, skin damage from burns, skin damage from ultraviolet light, and inflammatory and immune system diseases including retinopathy, ischemic dementia, Alzheimer's dementia, spinal cord injury, Parkinson's disease, and central nervous system disease.

A cosmetic composition containing an active ingredient or exosomes secreted from mesenchymal-like stem cells prepared by the preparation method according to an example embodiment may be provided.

The human pluripotent stem cells-derived mesenchymal-like stem cells according to the present disclosure have enhanced anti-inflammatory efficacy, immunosuppression and tissue regeneration ability compared to bone marrow-derived mesenchymal stem cells.

### Example 1: Isolation and Culture of Mesenchymal-like Stem Cells with Enhanced Anti-Inflammatory Efficacy and Immunosuppression

FIG. 1 is a schematic diagram of a process of separating and culturing mesenchymal-like stem cells from human pluripotent stem cells according to an example embodiment. The human pluripotent stem cells were sub-cultured in passages lower than or equal to 70 passages after establishment of the cell line. The human pluripotent stem cells as sub-cultured in passages lower than or equal to 70 passages after establishment of the cell line were maintained and cultured in a 37°C and 5% CO₂ cell incubator. The maintained and cultured human pluripotent stem cells were isolated from the culture plate via simple enzyme treatment. After the formation of embryoid bodies, only cystic embryoid bodies were selected therefrom. Before coupling the cell-permeable 3D culture unit to a new culture plate (6 well plate), only for the first time during culture, 2 ml of the culture solution was first added to the new culture plate. Then, the cell-permeable 3D culture unit was coupled to the culture plate containing 2 ml of the culture solution. Thereafter, 2 ml of the culture solution was additionally added to the cell-permeable 3D culture unit, and the selected cystic embryoid bodies were loaded onto the coupled cell-permeable 3D culture unit. EGM-2MV was used as the culture medium placed in the new culture plate and the culture medium in the cell-permeable 3D culture unit. The embryoid bodies were cultured without changing the culture medium in the cell-permeable 3D culture unit loaded with the cystic embryoid bodies for two days. Thereafter, the culture solution was removed therefrom, and 4 ml of the culture solution was added only into the cell-permeable 3D culture unit. The culture medium was changed everyday to induce differentiation thereof into the mesenchymal-like stem cells.

### Example 2: Isolation and Proliferation of Mesenchymal-like Stem Cells with Enhanced Anti-Inflammatory Efficacy and Immunosuppression

The mesenchymal-like stem cells isolated in Example 1 passed through the cell-permeable 3D culture unit and moved toward the bottom of the cell-permeable 3D culture unit, such that cells in the form of clusters were isolated. Among the separated clusters, the multilayer-shaped cluster was mechanically removed. Among the separated clusters, only the monolayer-shaped cluster was cut into a size of 500 µm or smaller in a longitudinal direction and 500 µm or smaller in a transverse direction using a micropipette tip in a uniform manner and was selectively cultured. More preferably, the monolayer-shaped cluster was uniformized to 100 µm to 500 µm in a longitudinal dimension and 100 µm to 500 µm in a transverse dimension to culture the mesenchymal-like stem cells. After incubation for 5 to 7 days, only mesenchymal-like stem cells protruding from the cluster in the form of single cells were isolated, and sub-cultured to proliferate the cells. Before single cell separation, cells in the shape of clusters and epithelial cells that were not of the single cell type were completely removed with a micropipette tip. The isolated single cells were transferred to a new culture plate to induce proliferation thereof via sub-culture. In this connection, EGM-2MV was used as the culture medium.

Specifically, as shown in FIG. 4, when the clustered mesenchymal-like stem cells migrated toward the bottom of the cell-permeable 3D culture unit, the cell-permeable 3D culture unit was separated from the culture plate. After obtaining the mesenchymal-like stem cells in the form of clusters that have moved toward the bottom of the separated cell-permeable 3D culture unit, the multilayer-shaped clusters were mechanically removed, and the size of the monolayer-shaped clusters was uniformized and the size-uniformized monolayer-shaped clusters were selectively cultured. The mesenchymal-like stem cells proliferated from the monolayer-shaped clusters maintained their morphology and proliferation ability even under continuous sub-culture, such that no negative changes on the morphology and proliferation of cells were observed.

### Example 3: Analysis of Surface Antigen Expression of Mesenchymal-like Stem Cells

In order to characterize mesenchymal-like stem cells prepared according to the Examples 1 and 2, the expression of stem cell specific markers was analyzed using a flow cytometer (Fluorescence Activated Cell Sorting, FACS).

Specifically, proliferation-induced mesenchymal-like stem cells were single-celled using TrypLE and were suspended in phosphate buffered saline (PBS) at a concentration of 5 × 10⁵ cells/ml. Sox2, CD44, CD73, CD90, CD105, CD146, NG2, HLA-ABC, CD11b, CD11c, CD14, CD19, CD34, CD45, CD40, CD40L, CD80, CD86, CD95, CD133, KDR, Flt-1 , Tie-2, HLA-DR, Oct3/4, Tra-1-81 and Tra-1-60 antibodies were added to each of the cells. Reaction occurred at room temperature for 45 minutes. Then, flow cytometry was performed on each cell. The nuclear internal proteins SOX2 and Oct3/4 were treated with 0.1% triton X-100 for 5 minutes at room temperature to induce a cell membrane permeabilization process. Then, the antibody was added and flow cytometry was performed. The results are shown in Table 1 and FIG. 6 below.

**Table 1. Surface antigen expression level of mesenchymal-like stem cells**

| Marker | SOX2 | CD44 | CD73 | CD90 | CD 105 | CD146 | NG2 |
|---|---|---|---|---|---|---|---|
| Expression level (%) | 99.4 | 98.8 | 96.77 | 98.2 | 95.6 | 92.7 | 68.7 |
| Marker | HLA-ABC | CD11b | CDllc | CD14 | CD19 | CD34 | CD45 |
| Expression level (%) | 58.8 | 0.18 | 0.11 | 0.17 | 0.09 | 1.46 | 0.15 |
| Marker | CD40 | CD40L | CD80 | CD86 | CD95 | CD133 | KDR |
| Expression level (%) | 0.45 | 0.05 | 1.22 | 0.04 | 10.3 | 0.16 | 0.26 |
| Marker | Flt-1 | Tie-2 | HLA-DR | Oct3/4 | Tra-1-81 | Tra-1-60 | |
| Expression level (%) | 4.46 | 0.98 | 0.08 | 0.2 | 0.03 | 0.08 | |

Referring to Table 1 and FIG. 6, the mesenchymal-like stem cells prepared by the preparation method according to the present disclosure expressed all of CD44, CD73, CD90, and CD105 as mesenchymal stem cell-specific markers at a level of 95% or greater. To the contrary, CD45, CD34, CD14, and CD19 were expressed from the mesenchymal-like stem cells prepared by the preparation method according to the present disclosure at 2% or less level. Further, the expression of the undifferentiated control markers Oct3/4, Tra-1-60, Tra-1-81 and the immune rejection antigen HLA-DR was hardly observed. Therefore, it was identified that the mesenchymal-like stem cells prepared by the preparation method according to the present disclosure have all of the characteristics of mesenchymal stem cells as defined by the International Society for Cell Therapy. Further, SOX2 and CD146 which are not defined as a characteristic of adult mesenchymal stem cells by the International Society for Cell Therapy was expressed from the mesenchymal-like stem cells prepared by the preparation method according to the present disclosure at a level of 90% or greater. CD95 was identified as being expressed from the mesenchymal-like stem cells prepared by the preparation method according to the present disclosure at a significantly lower level. Because the mesenchymal-like stem cells as prepared in the present disclosure are obtained by inducing of differentiation of human pluripotent stem cells of passage 70 or lower, the mesenchymal-like stem cells may be predicted to have the surface antigen expression characteristics shown in Table 1. In addition, because only selected cystic embryoid bodies among embryoid bodies that have obtained by inducing of differentiation of the human pluripotent stem cells are separated using the cell-permeable 3D culture unit, and the monolayer-shaped cell clusters are uniformized in a size and are cultured, the surface antigen expression characteristics in Table 1 are kept constant. In particular, the mesenchymal-like stem cells prepared by the preparation method according to the present disclosure express SOX2 at 90% level or greater, and are different from conventional mesenchymal stem cells.

### Example 4: Analysis of Differentiation Ability of Mesenchymal-like Stem Cells

In order to identify the multi-differentiation ability of mesenchymal-like stem cells prepared through the Examples 1 and 2, the differentiations thereof into osteogenic cell, adipogenic cells, chondrogenic cells and myogenic cells as defined as characteristics of mesenchymal stem cells by the International Society for Cell Therapy were induced.

Specifically, the prepared mesenchymal-like stem cells were cultured in low-concentration glucose DMEM medium containing therein 10% FBS, 5 µg/ml insulin, 1 µM dexamethasone, 0.5 mM isobutylmethylxanthine and 60 µM indomethacin. Further, the cells were cultured in a low-concentration glucose DMEM medium containing therein 10% FBS, 1 µM dexamethasone, 10 mM β-glycerophosphate, and 60 µM ascorbic acid-2-phosphate. Then, whether the mesenchymal-like stem cells differentiate into osteogenic cells, adipogenic cells, and chondrogenic cells was checked. Whether the mesenchymal-like stem cells differentiate into the osteogenic cells was checked using Alizarin red S staining. Whether the mesenchymal-like stem cells differentiate into the adipogenic cells was checked using Oil Red O staining. Further, whether the mesenchymal-like stem cells differentiate into the chondrogenic cells was checked using Alcian blue staining.

In addition, in order to identify differentiation thereof into myogenic cells, the prepared mesenchymal-like stem cells were cultured in DMEM medium containing 20% FBS, 1% non-essential amino acid, 1% penicillin sulfate, and 0.1 mM β-mercaptoethanol. It was identified that in the differentiated myogenic cells, the smooth muscle-specific marker αSMA was expressed.

Therefore, as shown in FIG. 7, the mesenchymal-like stem cells prepared according to the preparation method of the present disclosure exhibit a certain differentiation ability into the osteogenic cells, adipogenic cells, chondrogenic cells, and myogenic cells, and thus satisfy the characteristics of mesenchymal stem cells suggested by the World Stem Cell Therapy Society.

### Example 5: Analysis of Proliferation Ability and Cell Size of Mesenchymal-like Stem Cells

To identify the proliferation ability of mesenchymal-like stem cells produced through the Examples 1 and 2, the total number of cell divisions from 3 passage to 7 passage was identified. The total number of cell divisions was compared with that of bone marrow-derived mesenchymal stem cells, which are adult stem cells. The results are shown in Table 2 and FIG. 8 below.

**Table 2. Proliferation ability of mesenchymal-like stem cells**

| Cell proliferation ability | Number of repetitions of test | Average (times) | Min (times) | Max (times) | Standard deviation |
|---|---|---|---|---|---|
| Mesenchymal-like stem cells | 3 | 16.13 | 15.82 | 16.665 | 0.46 |
| Bone marrow-derived mesenchymal stem cells | 3 | 10.17 | 10.06 | 10.26 | 0.10 |

As shown in Table 2 and FIG. 8, for mesenchymal-like stem cells, one cell divided an average of 16.13 times from 3 passage to 7 passage. To the contrary, for bone marrow-derived mesenchymal stem cell, one cell divided an average of 10.17 times from 3 passage to 7 passage. Therefore, it can be seen that mesenchymal-like stem cells are excellent in proliferation ability.

Based on a result of examining the cell size of proliferated mesenchymal-like stem cells, it was identified that the cell size of mesenchymal-like stem cells averaged 13.6 ± 0.3 µm as shown in Table 3 and FIG. 6. To the contrary, for bone marrow-derived mesenchymal stem cells, the cell size averaged 17.7 ± 0.4 µm. Therefore, it was identified that the size of mesenchymal-like stem cell was relatively small compared to that of bone marrow-derived mesenchymal stem cell. Since the size of mesenchymal-like stem cells is relatively smaller, the incidence of pulmonary embolism due to vascular obstruction may be lowered when the composition as the cell therapeutic agent is injected into a blood vessel. Therefore, the mesenchymal-like stem cells according to the present disclosure may be applied to a therapeutic composition including a cellular therapeutic composition, a cellular gene therapeutic composition, a tissue engineering therapeutic composition, an anti-inflammatory therapeutic composition, an immune therapeutic composition, and a composition for preventing or treating cancer, thereby increasing the efficiency of treatment and reducing the likelihood of side effects.

**Table 3. Cell size of mesenchymal-like stem cells**

| Cell size | Number of repetitions of test | Average (µm) | Min (µm) | Max (µm) | Standard deviation |
|---|---|---|---|---|---|
| Mesenchymal-like stem cells | 3 | 13.6 | 13.4 | 13.9 | 0.3 |
| Bone marrow-derived mesenchymal stem cells | 3 | 17.7 | 17.3 | 18.1 | 0.4 |

### Example 6: High Purity Isolation of Mesenchymal-like Stem Cells

The mesenchymal-like stem cells prepared through the Examples 1 and 2 were identified as high-purity cells free of undifferentiated cells. That is, the expression of Oct4 as an undifferentiated control marker for mesenchymal-like stem cells in maintenance and proliferation was identified through qPCR. Further, human embryonic stem cell lines which are undifferentiated pluripotent stem cells were compared with human skin fibroblast cells (hFF). As a result, as shown in FIG. 9, Oct4 as an undifferentiated regulatory marker was expressed only in human embryonic stem cells. It could be identified that in the mesenchymal-like stem cells prepared according to the preparation method of the present disclosure, undifferentiated cells were not mixed therein, as in the human skin fibroblast cells (hFF). Mesenchymal-like stem cells prepared according to the preparation method of the present disclosure may be identified as cells isolated at high purity without detection of undifferentiated cells thereof.

### Example 7: Genetic Safety Analysis of Mesenchymal-like Stem Cells

Chromosome analysis and single nucleotide polymorphism (SNP) analysis were performed to identify the genetic safety of mesenchymal-like stem cells.

Specifically, for chromosome analysis, mesenchymal-like stem cells were added in a 10 ml medium containing 20 µl of a colchicine solution at a concentration of 100 µg/µl, and then left at 37°C for 2 hours. Then, after centrifugation at 500 rpm for 5 minutes, the supernatant was removed. Then, the cells were suspended in a 0.075M KCl stock solution, and then they were left in a constant temperature water bath at 37°C for 25 minutes. Thereafter, 5 drops of a fixing solution (methanol: glacial acetic acid = 3 : 1, v/v) were added thereto, followed by centrifugation at 1,200 rpm for 8 minutes. Thus, the supernatant was removed. The fixing solution was additionally added thereto, and the cells were left at room temperature for 10 minutes. This process was performed twice. Thereafter, a drop of the cell suspension was dropped onto a slide glass immersed in cold 70% ethanol. Then, the slide was dried using an alcohol lamp. Then, dyeing was performed for 12 minutes using 5% Gimesa solution. Then, the dyeing solution was removed via distillation, followed by drying in air, and then observation under an optical microscope. SNP analysis was entrusted to a specialized testing agency such that the presence or absence of chromosomal abnormalities was identified using the Illumina SNP chip. As shown in FIGS. 10A and 10B, chromosomes are normal in all mesenchymal-like stem cells prepared with three batch numbers. No SNP abnormality was observed. Therefore, the mesenchymal-like stem cells prepared according to the preparation method of the present disclosure proved to be genetically stable.

### Example 8: Tissue Regeneration Analysis of Mesenchymal-like Stem Cells

Various analyzes were performed to evaluate the functionality of mesenchymal-like stem cells prepared through the Examples 1 and 2. In order to analyze tissue regeneration, an In vitro Cell Migration Assay was performed. In tissue regeneration analysis, bone marrow-derived mesenchymal stem cells were used as control cells.

Specifically, mesenchymal-like stem cells and bone marrow-derived mesenchymal stem cells were dispensed at a concentration of 3 × 10⁴ cells/well by 70 µl each µ-dish 35mm, and were incubated for 24 hours in a 37°C, 5% CO₂ incubator. Thereafter, the culture unit was removed. 2 ml of 10% DMEM medium was added thereto. Further, while observing under a microscope, the number of migration of cells was measured.

As shown in FIG. 11, the migration ability of the prepared mesenchymal-like stem cells was averaged 67.51%. It is shown that the migration ability of the mesenchymal-like stem cells prepared according to the preparation method of the present disclosure is 350% or higher than that of bone marrow-derived mesenchymal stem cells. In other words, it is confirmed that mesenchymal-like stem cells prepared by one embodiment of the present disclosure are capable of rapid cell migration and proliferation when administered in vivo. Therefore, it may be predicted that the prepared mesenchymal-like stem cells have considerably higher tissue regeneration ability.

### Example 9: Analysis of Exchange-of-Materials Ability and Angiogenesis Ability of Mesenchymal-like Stem Cells

The exchange-of-materials ability and angiogenesis ability were analyzed in another approach for functional evaluation of mesenchymal-like stem cells prepared in Examples 1 and 2.

Specifically, the exchange-of-materials ability was analyzed by utilizing calcein as a fluorescent material that may move between a gap and a junction and which is used to analyze the interaction between cells. First, calcein staining was performed on human umbilical vein endothelial cells (HUVEC) as one of the vascular cells. Dil-labeled mesenchymal-like stem cells were co-cultured therewith for 1 hour in an incubator at 37°C. The co-culture was analyzed under a fluorescence microscope. As shown in FIG. 12, we may identify the rapid material transfer between the HUVEC cells indicated by fluorescence and the Dilmesenchymal-like stem cells indicated by red via the co-culture. Based on a result of flow cytometry after 48 hours, the number of cells with exchange-of-materials was identified as 46.15%. In other words, the mesenchymal-like stem cells prepared according to the preparation method of the present disclosure may promote interactions between cells through rapid gap-junction binding with blood vessel cells, thereby supplying useful substances.

For the analysis of angiogenesis ability, Matrigel thawed at room temperature was dispensed into a 96 well plate by 50 µl. Then, after standing at 37°C for 30 minutes, single-celled mesenchymal-like stem cells 1 to 2 × 10⁴ cells were seeded on the Matrigel, and cultured at 37°C for 24 hours. Then, 10% formaldehyde fixing solution was added thereto and the cells were further incubated for 10 minutes. Then, under a 10-fold microscope, random observations of 3 to 5 sites were carried out to observe spontaneous angiogenesis (spontaneous tubule formation). As shown in FIG. 13, we may identify that all three batch numbers of mesenchymal-like stem cells had the spontaneous angiogenesis on Matrigel. This indicates that mesenchymal-like stem cells prepared in Examples 1 and 2 possess spontaneous angiogenesis ability. In other words, the characteristics indicate that the mesenchymal-like stem cells prepared according to the preparation method of the present disclosure have the ability to promote angiogenesis and exchange-of-materials at the site of injury.

### Example 10: Analysis of Immune Cell Proliferation Inhibition and Anti-Inflammatory Efficacy of Mesenchymal-like Stem Cells

In analyzing one of the functions of mesenchymal-like stem cells prepared in Examples 1 and 2, the cell proliferation inhibition and expression of anti-inflammatory related proteomes were analyzed via co-culture thereof with immune cells.

Specifically, 1,000, 2,000, 5,000 and 10,000 mesenchymal-like stem cells prepared according to Examples 1 and 2, and 1,000, 2,000, 5,000 and 10,000 bone marrow-derived mesenchymal stem cells were prepared, respectively. Further, peripheral blood mononuclear cell (PBMC) 2 × 10⁵ cells/well were co-cultured therewith in a 96 well plate for 5 days. Each experimental group was labeled with carboxy fluorescein succinimidyl ester (CFSE) to measure the ability of inhibition of PBMC proliferation (%). The results are shown in Table 4 and FIG. 14 below.

**Table 4. Ability to inhibit immune cell proliferation due to increase in number of mesenchymal-like stem cells when co-cultured with PBMC**

| Test group | Mesenchymal-like stem cells | Average (%) | Min (%) | Max (%) | Standard deviation |
|---|---|---|---|---|---|
| 1 | 1,000 | 2.54 | 2.55 | 3.26 | 0.64 |
| 2 | 2,000 | 4.55 | 4.55 | 4.75 | 0.24 |
| 3 | 5,000 | 13.23 | 13.23 | 13.91 | 0.87 |
| 4 | 10,000 | 36.53 | 36.53 | 36.92 | 0.47 |

| | | | | | |
|---|---|---|---|---|---|
| *PBMC: 2 × 10⁵ cells/well | | | | | |

As shown in Table 4 and FIG. 14, mesenchymal-like stem cells may be identified to inhibit the proliferation of PBMC in a concentration-dependent manner. In other words, mesenchymal-like stem cells according to the present disclosure have significantly higher anti-proliferative effects than bone marrow-derived mesenchymal stem cells have. Therefore, immunosuppression of the mesenchymal-like stem cells according to the present disclosure is excellent.

The expression of genes related to anti-inflammatory action and immunosuppression was analyzed through genome and proteome analysis of mesenchymal-like stem cells. As a result, it was identified that Gata3, Adora2a and Gps2 which typically exhibit anti-inflammatory actions were expressed from the mesenchymal-like stem cells according to the present disclosure at a level higher by about 11 times to about 100 times or greater than from bone marrow-derived mesenchymal stem cells. Therefore, the mesenchymal-like stem cells have superior anti-inflammatory efficacy and immunosuppression compared to bone marrow-derived mesenchymal stem cells.

### Example 11: Ability to Secrete Functional Useful Substances from Mesenchymal-like Stem Cells

For functional analysis of mesenchymal-like stem cells prepared in Examples 1 and 2, the concentration of useful substances secreted therefrom was measured. Specifically, prepared mesenchymal-like stem cells were cultured for 24 hours in serum-free DMEM medium. Then, centrifugation thereof was performed at 500 rpm, and only a top medium was recovered. The recovered medium was quantitatively analyzed in terms of MMP-1, HGF and CD95 using enzyme-linked immunosorbent assay (ELISA). For comparison with adult stem cells, bone marrow-derived mesenchymal stem cells were compared therewith. The results are shown in FIG. 15.

As shown in FIG. 15, the concentration of MMP-1 as a representative tissue regeneration-related protein as expressed in mesenchymal-like stem cells was 20,953 pg/ml. The concentration of MMP-1 as a representative tissue regeneration-related protein as expressed in bone marrow-derived mesenchymal stem cells was 1,237 pg/ml. That is, MMP-1 was secreted from the mesenchymal-like stem cells at a level higher by about 16 times than from bone marrow-derived mesenchymal stem cells. The concentration of hepatocyte growth factor (HGF) as a proteome related to cell growth and proliferation expressed from mesenchymal-like stem cells was 1,360 pg/ml, and the concentration of the HGF as a proteome related to cell growth and proliferation expressed from bone marrow-derived mesenchymal stem cells was 534 pg/ml. In other words, HGF was secreted from mesenchymal-like stem cells at a level higher by about 2.5 times or greater than from the bone marrow-derived mesenchymal stem cells. On the other hand, the concentration of CD95 as known to play an important role in inducing apoptosis, as expressed in mesenchymal-like stem cells was 53 pg/ml, while the bone marrow-derived mesenchymal stem cells express the CD95 of 800 pg/ml. Therefore, CD95 was expressed from mesenchymal-like stem cells at a level lower by 15 times or greater than from bone marrow-derived mesenchymal stem cells. Therefore, mesenchymal-like stem cells prepared according to the present disclosure have superior ability to secrete proteins related to tissue regeneration and cell proliferation compared to existing bone marrow-derived mesenchymal stem cells. Further, the apoptosis-inducing protein is expressed therefrom at a remarkably low level, the ability of tissue regeneration and cell viability thereof is very good.

### Example 12: Identification of Gene Expression Characteristics related to Inflammation Regulation and Immunosuppression of Isolated and Cultured Mesenchymal-like Stem Cells

Next Generation Sequence (NGS) was performed on mesenchymal-like stem cells as isolated and cultured in Examples 1 and 2, and bone marrow-derived mesenchymal stem cells, and then the difference in gene expression therebetween was analyzed based on the database published by NCBI GEO.

Specifically, RNA sequencing (RNA-seq) was performed to compare gene expression of mesenchymal-like stem cells with gene expression of bone marrow-derived mesenchymal stem cells. The former and latter cells were dispensed in a 100 mm dish at a density of 3 × 10⁵ cells/dish and were used for analysis at the time of confluence 80%. Total RNA of cells was extracted for RNA-seq. All RNA samples were identified as having a uniform quality above a reference. The cDNA library was prepared according to the standard procedure of the TruSeq Stranded mRNA LT Sample Prep Kit (Illumina). The cDNA library was sequenced on NovaSeq 6000 System (Illumina) according to TruSeq Stranded mRNA Sample Preparation Guide (Part #15031047 Rev. E). Each RNA-seq data was generated using 3 samples per cell (n = 3). Phred quality score was calculated using BBDuk (BBtools) from the obtained Illumina read data. Only data with average Q30 or higher among the total data were used. Selected read data were mapped onto a reference genome sequence (hg19; genome database: USCS) using Bowtie2 (Langmead & Salzberg, 2012). Bedtools (https://bedtools.readthedocs.io/en/latest/) was used to calculate the read data. Mapping and quantification were performed on each sample. The quantified gene expression information was quantile normalized using edgeR (Robinson, McCarthy, & Smyth, 2010). All data were analyzed for comparison between mesenchymal-like stem cells and bone marrow-derived mesenchymal stem cells. The results are shown in FIGS. 16 and 17.

As shown in FIG. 16, it was identified based on the analysis of the inflammation regulation gene that the genes that were expressed from mesenchymal-like stem cells at a level higher by about 2 times or greater than from the bone marrow-derived mesenchymal stem cells were 16 kinds of genes including Gata3, Adora2a, Gps2, Psmal, Pbk, Lrfn5, Cdh5, Apoe, Foxfl, Tek, Cxcl1, Ptger4, Acp5, Bcr, Socs5, and Mdk. In particular, Gata 3 gene was expressed at about 108 times higher level, Adora2a gene was expressed at about 27.97 times higher level, and Gps2 gene was expressed at about 11.02 times higher level. Therefore, it was identified that the inflammation regulating gene was expressed from mesenchymal-like stem cells at a level higher by 10 times or greater than from bone marrow-derived mesenchymal stem cells.

The results of analyzing gene expression related to immunosuppression through analysis using RNA-seq are shown in FIG. 17. The genes that were expressed from mesenchymal-like stem cells at a level higher by about 2 times or greater than from the bone marrow-derived mesenchymal stem cells were 11 kinds of genes including Gata3, Gps2, Psmal, Apoe, Foxfl, Tek, Cxcl1, Ptger4, Bcr, Socs5, and Mdk. In particular, the Gata 3 gene was expressed at about 108 times higher level and the Adora2a gene was expressed at about 11.02 times higher level. Thus, the gene related to immunosuppression was expressed from mesenchymal-like stem cells at a level higher by 10 times or greater than from bone marrow-derived mesenchymal stem cells.

### Example 13: Proteome Analysis of Cell Lysate and Culture Supernatant from Isolated and Cultured Mesenchymal-like Stem Cells

The cell lysate and the medium of mesenchymal-like stem cells prepared in Examples 1 and 2 were analyzed in terms of increase and decrease in proteomes using L507 antibody array.

Specifically, to compare the protein composition of the cell lysate and culture supernatant of mesenchymal-like stem cells with those of bone marrow-derived mesenchymal stem cells, a semi-quantitative protein antibody array chip L507 (RayBiotech) was used. Cell lysate and culture supernatant were obtained together at the time point of obtaining total RNA. The L507 chip may be used for analyzing 507 kinds of proteins based on biotin labeling, and was selected for protein analysis of mesenchymal-like stem cells. For analysis, protein of cell lysate and culture supernatant was extracted. Further, the protein was quantified using the BCA protein assay kit (Abcam) (50 to 200 µg range). The quantified protein was dispensed in the same amount and labeled with biotin. Then, protein hybridization was performed. A fluorescence image by the hybridization was visualized using streptavidin-cyanine3 conjugate, and was scanned with GenePix 4100A Microarray Scanner (Molecular Devices). All data were quantile normalized using edgeR and then was analyzed using GenePix Pro 7.0 software (Molecular Devices). All data were analyzed for comparison between mesenchymal-like stem cells and bone marrow-derived mesenchymal stem cells. The results are shown in FIGS. 18 to 21.

As shown in FIG. 18, lysate proteomes of the mesenchymal-like stem cells are analyzed. Mesenchymal-like stem cells expressed Angiopoietin-4 (ANGPT4), bone morphogenic protein receptor (BMPR)-1B, human chemokine receptor (HCR), Activin B, Activin RII, CCR1, intracellular adhesion molecule 2 (ICAM-2) at a level higher by at least 2 times than the bone marrow-derived stem cells expressed the same. To the contrary, as shown in FIG. 19, Mesenchymal-like stem cells expressed 15 kinds of proteomes including Angiogenin (ANG), Angiopoetin-1 (ANG-1), Angiopoetin-2 (ANG-2), Bone morphogenic proteins receptorlA (BMPR1A), and CXC-chemokine receptor6 (CXCR6) at a level lower by at least 2 times than the bone marrow-derived mesenchymal stem cells expressed the same.

As shown in FIG. 20, the proteome for culture supernatant was analyzed. Mesenchymal-like stem cells secreted GRO-a, IL-15R alpha, FasL, Activin RII, BMP-2, CCR2, CXCL14, and FGFR4 at a level higher by at least 2 times or greater than the bone marrow-derived stem cells did. As shown in FIG. 21, mesenchymal-like stem cells expressed tissue inhibitor of methalloproteinases-2 (TIMP-2), ActivinA, vascular endothelial factor (VEGFA), Follistantin-like 1 (FSTL1), ErB4, and Thrombospondin-1 at a lower level.

While a few example embodiments have been shown and described with reference to the accompanying drawings, it will be apparent to those skilled in the art that various modifications and variations can be made from the foregoing descriptions. For example, adequate effects may be achieved even if the foregoing processes and methods are carried out in different order than described above, and/or the aforementioned elements, such as systems, structures, devices, or circuits are combined or coupled in different forms and modes than as described above or be substituted or switched with other components or equivalents.

Thus, other implementations, alternative embodiments and equivalents to the claimed subject matter are construed as being within the appended claims.

## Claims

1. A method of preparing mesenchymal-like stem cells, the method comprising:
(a) preparing human pluripotent stem cells cultured to passage 70 or lower after establishment of cell lines;
(b) inducing differentiation of the human pluripotent stem cells to produce embryoid bodies and selecting cystic embryoid bodies therefrom;
(c) loading the cystic embryoid bodies on a cell-permeable three-dimensional (3D) culture unit to isolate mesenchymal-like stem cells therefrom;
(d) isolating only monolayer-shaped cell clusters from the mesenchymal-like stem cells passing through the cell-permeable 3D culture unit; and
(e) uniformizing each of longitudinal and transverse sizes of the monolayer-shaped cell clusters to 100 µm to 500 µm, and culturing the mesenchymal-like stem cells,
wherein the mesenchymal-like stem cells have anti-inflammatory efficacy and immunosuppression, and express CD90 and SOX2 at a level of 95% or greater.

2. The method of claim 1, wherein the cell-permeable 3D culture unit is made of at least one selected from a group consisting of nylon, fiber, polyethylene, polypropylene, graphene, titanium, copper, nickel, silver, gold and platinum.

3. Mesenchymal-like stem cells derived from human pluripotent stem cells prepared by the method of claim 1, wherein the mesenchymal-like stem cells express MMP-1 protein and HGF protein at a higher level than bone marrow-derived mesenchymal stem cells express MMP-1 protein and HGF protein, express CD95 at a lower level than the bone marrow-derived mesenchymal stem cells express CD95, and express CD90 and SOX2 at a level of 95% or greater.

4. The mesenchymal-like stem cells of claim 3, wherein the mesenchymal-like stem cells express the MMP-1 protein at a level higher by at least 15 times than bone marrow-derived mesenchymal stem cells express the MMP-1 protein, wherein the mesenchymal-like stem cells express the HGF protein at a level higher by at least 2 times than bone marrow-derived mesenchymal stem cells express the HGF protein.

5. The mesenchymal-like stem cells of claim 3, wherein the mesenchymal-like stem cells express CD95 at a level lower by at least 15 times than bone marrow-derived mesenchymal stem cells express CD95.

6. The mesenchymal-like stem cells of claim 3, wherein the mesenchymal-like stem cells express an inflammation-regulating gene at a level higher by 2 times to 100 times or greater than bone marrow-derived mesenchymal stem cells express the inflammation-regulating gene, and
the inflammation-regulating gene comprises at least one selected from a group consisting of Gata3, Adora2a, Gps2, Psmal, Pbk, Lrfn5, Cdh5, Apoe, Foxfl, Tek, Cx3cl1, Ptger4, Acp5, Bcr, Socs5, and Mdk.

7. The mesenchymal-like stem cells of claim 3, wherein the mesenchymal-like stem cells co-express Gata3, Adora2a, and Gps2 genes.

8. The mesenchymal-like stem cells of claim 3, wherein the mesenchymal-like stem cells express an immunosuppression gene at a level higher by 2 times to 100 times or greater than bone marrow-derived mesenchymal stem cells express the immunosuppression gene, and
the immunosuppression gene comprises at least one selected from a group consisting of Gata3, Gps2, Psmal, Apoe, Foxfl, Tek, Cx3cl1, Ptger4, Bcr, Socs5, and Mdk.

9. The mesenchymal-like stem cells of claim 3, wherein the mesenchymal-like stem cells co-express Gata3, Gps2, and Psmal genes.

10. A therapeutic composition comprising the mesenchymal-like stem cells of claim 3, wherein the therapeutic composition comprises one selected from a group consisting of a cellular therapeutic composition, a cellular gene therapeutic composition, a tissue engineering therapeutic composition, an anti-inflammatory therapeutic composition, an immune therapeutic composition, and a composition for preventing or treating cancer.

11. The therapeutic composition of claim 10, wherein the therapeutic composition further comprises an active ingredient or exosomes secreted from the mesenchymal-like stem cells.

12. The therapeutic composition of claim 10 or 11, wherein the therapeutic composition prevents or treats at least one disease selected from a group consisting of multiple sclerosis, systemic sclerosis, acute myocardial infarction, chronic myocardial infarction, chronic lung disease, acute lung disease, Crohn's disease, fecal incontinence, graft versus host disease, lower extremity ischemia disease, Burger's disease, foot ulcer, lupus, rheumatoid arthritis, acute and chronic pyelitis, inflammatory cystitis, interstitial cystitis, underactive bladder, overactive bladder, frozen shoulder, rotator cuff injury and rupture, musculoskeletal injury caused by various movements, knee cartilage injury, tinnitus, atopic dermatitis, psoriasis, skin damage from burns, skin damage from ultraviolet light, and inflammatory and immune system diseases including retinopathy, ischemic dementia, Alzheimer's dementia, spinal cord injury, Parkinson's disease, and central nervous system disease.

13. A transporter comprising the mesenchymal-like stem cells of claim 3, wherein the transporter carries a pharmaceutical composition therein.

14. A composition for prevention or treatment of a disease, the composition comprising an active ingredient or exosomes secreted from the mesenchymal-like stem cells of claim 3.

15. The composition of claim 14, wherein the disease comprises at least one selected from a group consisting of multiple sclerosis, systemic sclerosis, acute myocardial infarction, chronic myocardial infarction, chronic lung disease, acute lung disease, Crohn's disease, fecal incontinence, graft versus host disease, lower extremity ischemia disease, Burger's disease, foot ulcer, lupus, rheumatoid arthritis, acute and chronic pyelitis, inflammatory cystitis, interstitial cystitis, underactive bladder, overactive bladder, frozen shoulder, rotator cuff injury and rupture, musculoskeletal injury caused by various movements, knee cartilage injury, tinnitus, atopic dermatitis, psoriasis, skin damage from burns, skin damage from ultraviolet light, and inflammatory and immune system diseases including retinopathy, ischemic dementia, Alzheimer's dementia, spinal cord injury, Parkinson's disease, and central nervous system disease.

16. A cosmetic composition comprising an active ingredient or exosomes secreted from the mesenchymal-like stem cells of claim 3.
